# FASCICULE DE BREVET EUROPEEN

(11) **EP 0 752 473 B1**
(45) Date de publication et mention de la délivrance du brevet: **05.11.2008**
(21) Numéro de dépôt: 96401445.0
(22) Date de dépôt: 28.06.1996
(51) Int. Cl.: C12N 15/31, C12N 15/01, C07K 14/205, C12N 1/20, G01N 33/569, G01N 33/577, A61K 39/106, A61K 39/40, C12Q 1/10, C12R 1/01, C12N 9/80

(54) **Clonage et caractérisation du gène flbA de H. pylori; production de souches aflagellées**
Klonierung und Karakterisierung des H. pylori flbA Gens: Produktion von flagellenlosen Stämmen
Cloning and characterization of H. pylori flbA gene: production of flagella-free strains

(30) Priorité: 04.07.1995 FR 9508068
(43) Date de publication de la demande: 08.01.1997
(60) Demande divisionnaire: 05025860.7
(73) Titulaire: INSTITUT PASTEUR, 75724 Paris Cedex 15 (FR); INSTITUT NATIONAL DE LA SANTE ET DE LA RECHERCHE MEDICALE (INSERM), 75654 Paris Cédex 13 (FR)
(72) Inventeur: Suerbaum, Sebastian, D-44787 Bochum (DE); Labigne, Agnès, D-91440 Bures sur Yvette (FR)
(74) Mandataire: Desaix, Anne

(56) Documents cités:
- EP-A- 0 329 570
- WO-A-93/07273
- FR-A- 2 669 929
- GUT, vol. 37, no. sup1, 7 - 9 Juillet 1995, page a62 XP000564512 SCHMITZ ET AL.: "The H. pylori flagellar biosynthesis regulatory protein FlbA affects the expression of flagellar components on the transcriptional level and is probably a membrane protein"
- AM. J. GASTROENTEROL., vol. 89, no. 8, Août 1994, page 1331 XP000564505 SUERBAUM ET AL.: "Cloning, sequencing and mutagenesis of the H. pylori flbA gene - a homolog of the lcrD/flbF/invA family of genes associated with motility and virulence"
- ABSTR. GEN. MEET. AM. SOC. MICROBIOL., vol. 95, 21 - 25 Mai 1995, page 181 XP000564454 SUERBAUM ET AL.: "Cloning, expression and mutagenesis of the H. pylori flbA gene - a homolog of the lcrD/flbF family of genes associated with motility and virulence"
- INFECT. IMMUN., vol. 61, no. 71, Juillet 1993, pages 2930-2936, XP002021624 MILLER ET AL.: "A Campylobacter jejuni homolog of the lcrD/flbF family of proteins is necessary for flagella biogenesis"
- J. BACTERIOL., vol. 173, no. 22, Novembre 1991, pages 7283-7292, XP002021625 RAMAKRISHNAN ET AL.: "The cell cycle-regulated flagellar gene flbF of Caulobacter crescentus is homologous to a virulence locus (lcrD) od Yersinia pestis"
- MOL. MICROBIOL., vol. 14, no. 4, 1994, pages 691-703, XP000564465 O'TOOLE ET AL.: "Non-motile mutants of Helicobacter pylori and Helicobacter mustelae defective in flagellar hook production"
- SOC. MICROB. ECOL. DIS., vol. 4(s), no. s1, Octobre 1991, page s136 XP002021626 FERRERO ET AL.: "Construction of urease deficient mutants of Helicobacter pylori by allelic exchange"

## Description

Helicobacter pylori (désigné également par l'expression H. pylori) est une bactérie à gram négatif retrouvée exclusivement à ce jour à la surface de la muqueuse de l'estomac chez l'homme.

Comme la plupart des bactéries, H. pylori est sensible à un milieu de pH acide mais cependant peut tolérer l'acidité en présence de taux physiologiques d'urée (Marshall et al (1990) Gastroenterol. 99: 697-702). En hydrolysant l'urée sous forme de dioxyde de carbone et d'ammoniac qui sont relargués dans le microenvironnement de la bactérie, l'uréase de H. pylori permet la survie de la bactérie dans l'environnement acide de l'estomac. Récemment des études menées sur des modèles animaux ont fourni des éléments suggérant que l'uréase est un facteur important dans la colonisation de la muqueuse gastrique (Eaton et al (1991) Infect. Immun. 59: 2470-2475). L'uréase est également suspectée de causer des dommages soit directement, soit indirectement, à la muqueuse gastrique.

Helicobacter pylori (H. pylori) est à l'heure actuelle reconnu comme l'agent étiologique des gastrites antrales, et apparait comme un des cofacteurs requis pour le développement des ulcères. Par ailleurs il semble que le développement de carcinomes gastriques puisse être associé à la présence de H. pylori.

Afin de mettre au point des moyens nouveaux sensibles et spécifiques pour la détection in vitro d'infections par des bactéries de l'espèce Helicobacter pylori, les inventeurs se sont intéressés au système de régulation de la mobilité de ces bactéries.

Dans ce but, ils se sont intéressés à différentes modifications des souches de H. pylori, modifications qui n'affectaient pas la reconnaissance de ces bactéries par des sérums de patients infectés mais qui néanmoins permettaient de ne pas obtenir de réaction de type « faux positif » en particulier avec des bactéries de la famille de Campylobacter et par exemple, Campylobacter jejuni.

Les inventeurs ont en outre constaté que les bactéries modifiées obtenues pouvaient être, le cas échéant, utilisées dans le cadre de la fabrication de compositions immunogènes ou vaccinantes. A cet égard, l'invention propose notamment des souches bactériennes vivantes atténuées.

Dans une première étape, les inventeurs ont identifié et isolé le gène flbA ayant une implication dans la régulation de la biosynthèse des flagelles de H. pylori et par conséquent, dans la régulation de la mobilité de la bactérie. La biosynthèse des flagelles comprend la synthèse des flagellines A et B, et de la gaine. Le gène flbA régule à la fois la synthèse des flagellines A et B, et la synthèse de la gaine contenant ces flagellines. Les inventeurs ont établi que le gène flbA était également intéressant en ce qu'il régulait la biosynthèse de la protéine d'ancrage de la bactérie, encore appelée "crochet".

La présente demande décrit donc une séquence nucléotidique du gène flbA régulant la biosynthèse des protéines des flagelles d'Helicobacter pylori, caractérisée en ce qu'elle est capable d'hybrider dans des conditions de forte stringence avec une sonde correspondant à un fragment de nucléotides de H. pylori amplifié à l'aide de deux oligonucléotides répondant aux séquences suivantes :
OLFlbA-1; ATGCCTCGAGGTCGAAAAGCAAGATG
OLFlbA-2 : GAAATCTTCATACTGGCAGCTCCAGTC, ou capable d'hybrider dans des conditions de forte stringence avec ces oligonucléotides.
Une telle séquence peut être obtenue par les étapes de :
- criblage d'une banque génomique contenant l'ADN chromosomique d'une souche H. pylori avec une sonde correspondant à un fragment de nucléotides de H. pylori amplifié à l'aide de deux oligonucléotides répondant aux séquences suivantes :
   OLFlbA-1 : ATGCCTCGAGGTCGAAAAGCAAGATG
   OLFlbA-2 : GAAATCTTCATACTGGCAGCTCCAGTC, ou capable d'hybrider dans des conditions de forte stringence avec ces oligonucléotides,
- récupération des séquences d'ADN hybridant avec ladite sonde,
- sous-clonage des séquences d'ADN obtenues dans un vecteur approprié de type plasmide et sélection de ceux des vecteurs modifiés hybridant dans des conditions de forte stringence avec la sonde correspondant au fragment d'ADN de H. pylori amplifié à l'aide des oligonucléotides OLFlbA-1 et OLFlbA-2
- séquençage des fragments d'ADN contenus dans les vecteurs plasmidiques hybridant avec la susdite sonde et détermination de la phase ouverte de lecture contenue dans ces fragments.

Avantageusement, on utilisera ces fragments d'ADN pour reconstituer la séquence codante du gène flbA correspondant à une phase ouverte de lecture comportant environ 2196 nucléotides.

La banque génomique contenant l'ADN chromosomique de H. pylori peut être obtenue à partir de toute souche de H. pylori. On peut aussi préparer une banque cosmidique à partir de l'ADN chromosomique de H. pylori.

Une souche utilisable pour la réalisation de cette banque est par exemple la souche N6 déposée à la NCIMB le 26 Juin 1992, sous le n° NCIMB40512.

Les deux oligonucléotides amorces utilisés pour préparer la sonde destinée à l'hybridation de l'ADN recherché présent dans la banque d'ADN de H. pylori sont choisis parmi les régions conservées des différentes protéines de la famille LcrD/FlbF.

Les deux oligonucléotides amorces OLFlbA-1 et OLFlbA-2 ont permis d'amplifier un fragment, utilisable comme sonde, de 130 paires de bases répondant à la séquence suivante :

ATG CCA GGA AAG CAA ATG GCG ATT GAT GCG GAT TTA AAT TCA GGG
CTT ATT GAT GAT AAG GAA GCT AAA AAA CGG CGC GCC GCT CTA AGC
CAA GAA GCG GAT TTT TAT GGT GCG ATG GAT GGC GCG TCT AAA TTT

Les conditions de forte stringence dont il est question ci-dessus sont les suivantes : l'hybridation est réalisée à 42°C en présence de 50% Formamide dans un tampon 2XSSC contenant 0,1% SDS (1XSSC correspond à 0,15 M NaCl plus 15 mM de citrate de sodium - pH 7,0). Les lavages sont réalisés avec 2XSSC plus 0,1% SDS à 68°C par exemple deux fois pendant une heure.

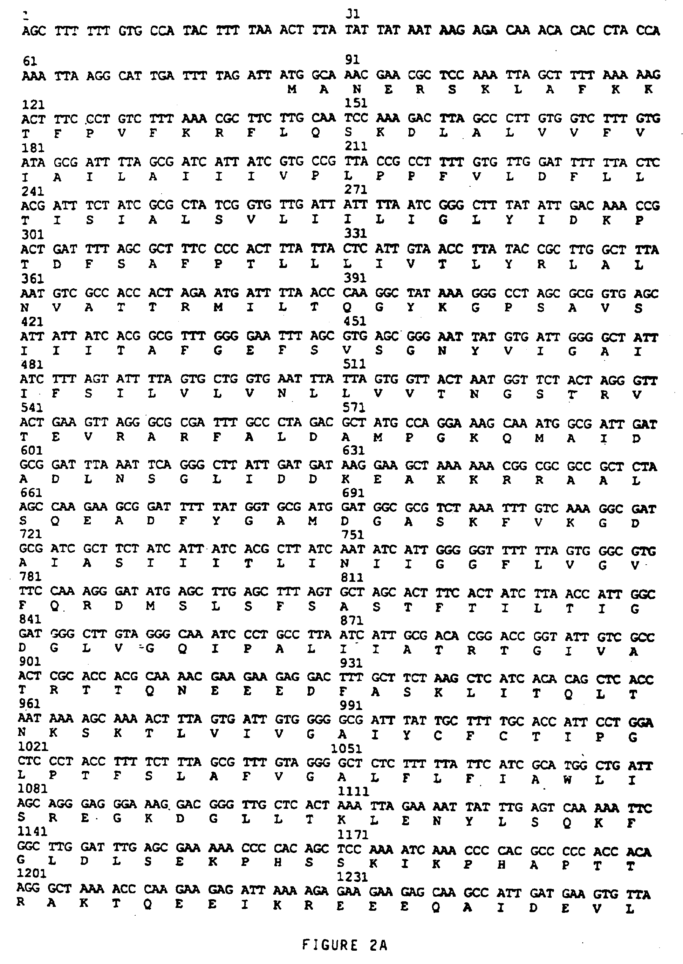

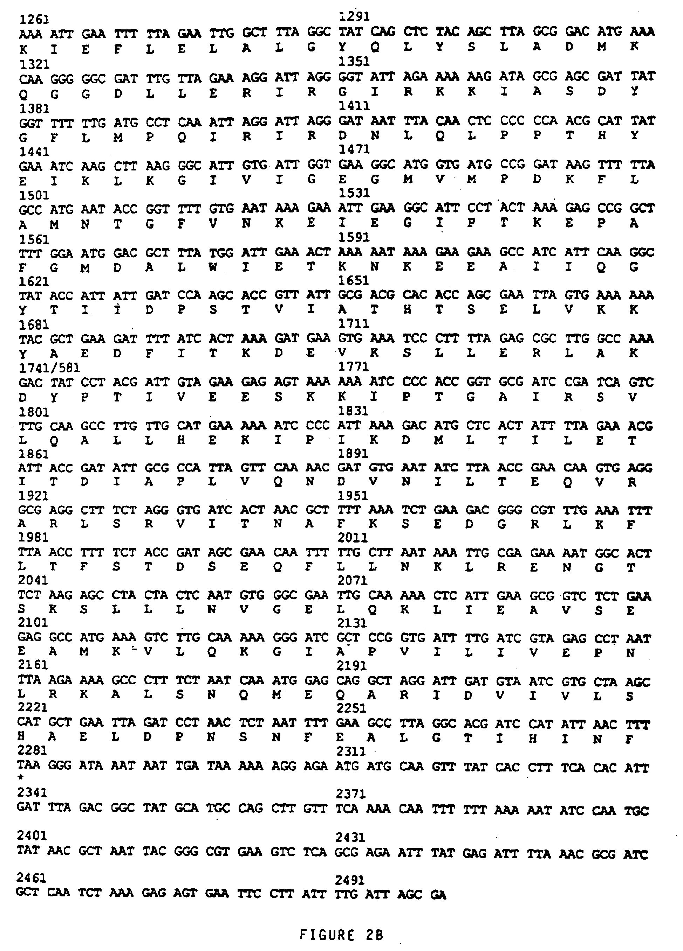

Une séquence nucléotidique particulièrement intéressante pour obtenir une souche bactérienne recombinante selon l'invention résulte de la mutation de la séquence du gène flbA correspondant à l'enchaînement de nucléotides représenté à la figure 2, ou codant pour la protéine représentée à la figure 2.

Une séquence nucléotidique, décrite dans la présente demande, est caractérisée en ce qu'elle code pour une protéine répondant à la séquence d'acides aminés représentée à la figure 2 ou pour une séquence d'acides aminés ayant les mêmes propriétés régulatrices vis à vis de la biosynthèse des protéines des flagelles de H. pylori, que la susdite séquence.

L'invention a pour objet l'utilisation pour la détection in vitro d'une infection par H. pylori dans un échantillon de fluide biologique prélevé chez un patient, d'une souche bactérienne recombinante d'Helicobacter pylori, ayant un phénotype aflagellé résultant de la mutation par délétion, substitution ou insertion de bases ou d'un fragment dans la séquence de nucléotides du gène flbA participant à la régulation de la biosynthèse des protéines des flagelles de H. pylori, ladite séquence nucléotidique du gène flbA hybridant dans des conditions stringentes avec une sonde correspondant à un fragment de nucléotides de H. pylori amplifié à l'aide des deux oligonucléotides OLFlba-1 et OLFlba-2, de façon telle que :
- soit le gène flbA n'est plus exprimé dans un hôte cellulaire,
- soit l'expression du gène flbA dans un hôte cellulaire ne permet pas la biosynthèse des flagellines A et B ni de la gaine les contenant et le cas échéant ne permet pas la synthèse de la protéine d'ancrage de H. pylori ou le crochet.

La modification subie par la séquence nucléotidique utilisée dans l'invention doit être telle qu'elle est irréversible et notamment qu'elle reste irréversible, lors de la recombinaison de cette séquence avec le gène flbA présent dans une bactérie que l'on transformerait avec une séquence nucléotidique ainsi modifiée. Cette recombinaison est par exemple de type « double crossing over ». De préférence, la modification de la séquence nucléotidique ne doit pas entraîner de modification substantielle - après remplacement par cette séquence modifiée du fragment correspondant du gène flbA normal dans une souche déterminée de H. pylori - des fonctions des gènes voisins.

La demande décrit aussi des séquences nucléotidiques constituant un fragment du gène flbA répondant aux critères précédents. A titre d'exemples, des fragments comportent au moins 6 nucléotides, de préférence au moins 50, voire au moins 100 nucléotides.

De tels fragments sont par exemple choisis soit pour leur caractère spécifique du gène flbA, soit pour leur appartenance à des régions conservées de plusieurs gènes codant pour des protéines de la famille LcrD/FlbF.

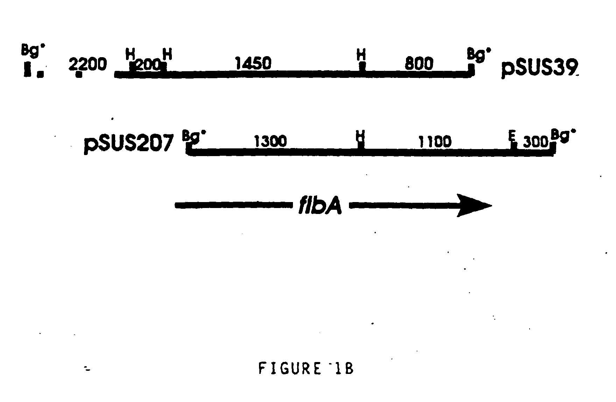

La demande décrit également les fragments du gène flbA limités par les sites de restriction présents sur le gène. Certains de ces sites sont définis à titre d'exemple à la figure 1B.

Un autre fragment décrit est un fragment d'au moins 1000 pb issu d'une quelconque région du gène flbA et comprenant de préférence un site de restriction ou susceptible d'accueillir un site de restriction.

D'autres séquences nucléotidiques décrites sont par exemple des acides nucléiques recombinants comprenant une séquence nucléotidique telle que celles qui ont été décrites ci-dessus, elle-même modifiée par insertion d'une cassette contenant un marqueur, par exemple un gène de résistance à un antibiotique ou un gène de résistance à un métal lourd tel que décrit dans la demande FR 9406202 déposée le 20/05194.

Une insertion peut ainsi être effectuée avec une cassette de résistance à la kanamycine. A cet égard, différentes techniques peuvent être mises en oeuvre et on se réferrera notamment à la publication de Labigne A. et al (J. of Bacteriology, vol. 170, 1988, p. 1704-1708) et à la publication de Labigne A. et al (Res. Microbiol 1992, 143, 15-26).

La demande décrit également des oligonucléotides spécifiques d'une séquence nucléotidique déterminée précédemment et caractérisés en ce qu'ils répondent à l'une des séquences suivantes :
OLFlbA-1: ATGCCTCGAGGTCGAAAAGCAAGATG
OLFlbA-2: GAAATCTTCATACTGGCAGCTCCAGTC
OLFlbA-7 CGGGATCCGTGGTTACTAATGGTTCTAC
OLFlbA-8: CGGGATCCTCATGGCCTCTTCAGAGACC

Selon une autre variante décrit une séquence d'acides aminés de la protéine FlbA de H. pylori est caractérisée en ce qu'elle est codée par une séquence nucléotidique telle que déterminée précédemment.

Une séquence d'acides aminés particulière de la protéine FlbA de H. pylori est présentée à la figure 2.

Ainsi, dans le cadre de l'invention, le gène flbA et la protéine exprimée par ce gène peuvent présenter un intérêt, en particulier pour l'utilisation dans des compostions immunogènes ou vaccinantes.

L'invention décrit donc des souches bactériennes d'Helicobacter pylori ayant un phénotype aflagellé résultant de la mutation par substitution, addition et/ou de la délétion de bases ou d'un fragment nucléotidique de la séquence nucléotidique définie ci-dessus du gène flbA participant à la régulation de la biosynthèse des protéines des flagelles de H pylori

La modification du gène flbA permet l'obtention d'une souche de type aflagellé c'est à dire n'exprimant plus les protéines FlaA et FlaB et de préférence n'exprimant plus les protéines de la gaine.

Selon une variante de réalisation de cette souche bactérienne, la souche obtenue sera en outre dépourvue de la protéine du crochet de H. pylori.

De façon préférée, une souche bactérienne répondant aux critères précédents est caractérisée en ce qu'elle est obtenue à partir de la souche N6 déposée à la NCIMB le 26 Juin 1992 sous le numéro NCIMB 40512.

A titre d'exemple, l'invention a pour objet une souche aflagellée recombinante de H. pylori, désignée par N6flbA- et déposée à la NCIMB le 30 Juin 1995 sous le n° NCIMB 40747.

Les souches aflagellées de H. pylori décrites dans la demande sont particulièrement intéressantes pour des utilisations en sérologie et par conséquent pour la détection in vitro d'une infection par H. pylori. Avantageusement, ces souches sont de type recombinantes

Ces souches présentent notamment l'avantage de permettre une détection in vitro spécifique et sensible de l'infection par H. pylori. En d'autres termes, l'invention permet avantageusement de détecter spécifiquement une infection par H. pylori en évitant en particulier les résultats « faux positifs » par exemple avec des souches de bactéries telles que Salmonella ou Campylobacter.

Etant donné que les souches de H. pylori de type aflagellé ainsi définies peuvent avoir également d'autres applications, et par exemple être mises en oeuvre dans le cadre de la préparation de compositions de vaccins, on pourra avoir intérêt à préparer des souches bactériennes recombinantes aflagellées ayant une seconde modification ou mutation et par exemple on pourra avoir recours à une souche bactérienne aflagellée caractérisée en ce qu'elle est en outre mutée de façon à produire une uréase atténuée, voire à ne plus produire d'uréase, la mutation consistant par exemple en une mutation de la séquence nucléotidique d'un ou plusieurs gènes choisis parmi les gènes ureA, ureB, ureC, ureD, ureE, ureF, ureG, ureH ou ureI. Les gènes de structure de l'uréase désignés par ureA, ureB, ureC et ureD de l'uréase ont été décrits dans la publication (Labigne et al (1991) J. Bacteriol. 173: 1920-1931). Les autres gènes ont été décrits dans la demande de brevet EP 0610322.

L'invention a pour objet des souches bactériennes recombinantes, caractérisées en ce qu'elles sont obtenues par mutation, par substitution, addition et/ou délétion de bases ou d'un fragment nucléotidique dans la séquence nucléotidique du gène flbA participant à la régulation de la biosynthèse des protéines des flagelles de H. pylori, ladite séquence hybridant dans des conditions stringentes avec une sonde correspondant à un fragment de nucléotides de H. pylori amplifié à l'aide des deux oligonucléotides dégénérés suivants : OLFlba-1 : ATGCCTCGAGGTCGAAAAGCAAGATG et OLFlba-2: GAAATCTTCATACTGGCAGCTCCAGTC, et ladite mutation étant telle que, soit le gène flbA n'est plus exprimé dans ladite souche bactérienne recombinante, soit le gène flbA ne permet pas la synthèse des flagelles de H. pylori, ladite mutation étant effectuée dans la souche N6 déposée à la NCIMB le 26 juin 1992 sous le numéro NCIMB40512.

Les souches bactériennes décrites dans la demande peuvent être utilisées telles quelles ou sous la forme d'extrait et en particulier un extrait bactérien total de souche obtenu à partir des souches décrites dans la demande.

Un tel extrait bactérien peut être préparé par extraction au N-OctylGlucoside. En ce cas, la technique de préparation utilisée est celle décrite par LELWALA-GURUGE J. (Scand. J. Infect Dis. 1992, 24: 457-465).

Un autre extrait bactérien peut être obtenu par extraction au PBS ou la glycine selon les techniques respectivement décrites par BAZILLOU M. et al (Clin. Diagn. Lab. Immuno., 1994, 1: 310-317) et AGUIRRE P.M. (Eur. J. Clin. Microbiol. Infect. Dis., 1992, 11: 634-639).

L'invention porte sur un extrait bactérien obtenu à partir des souches objet de l'invention obtenues à partir de la souche NCIMB40512 ou étant la souche NCIMB40747.

L'invention concerne une composition pour la détection in vitro d'une infection par H. pylori dans un échantillon de fluide biologique chez un patient, notamment dans un échantillon de sérum, comprenant à titre de principe actif, une souche bactérienne ou un extrait bactérien selon la description donnée dans la demande.

Les échantillons biologiques utilisés peuvent être de tous types et en particulier, il peut s'agir de tous fluides biologiques comme par exemple le sérum, la salive ou l'urine.

De même, les techniques mises en oeuvre pour la détection sont toutes les techniques faisant intervenir des réactions de type immunologique et en particulier de type antigène-anticorps. On utilise par exemple les techniques de type Western Blot, ELISA...

L'invention concerne donc également un procédé de détection in vitro d'une infection par H. pylori dans un échantillon de fluide biologique chez un patient, notamment dans un échantillon de sérum, comprenant les étapes de :
- mise en contact de l'échantillon testé avec une souche bactérienne définie dans la demande ou un extrait bactérien défini plus haut,
- détection d'une réaction immunologique entre ladite souche bactérienne et des anticorps dirigés contre H. pylori, présents dans l'échantillon testé.

A titre d'exemple, une détection in vitro sur un échantillon biologique pour rechercher une infection par H. pylori peut être réalisée en mettant en oeuvre les étapes suivantes : oeuvre les étapes suivantes:
- des plaques sont recouvertes de l'antigène utilisé pour la détection, qu'il s'agisse d'une protéine pure ou recombinante ou encore d'une souche aflagellée ou d'un extrait bactérien en particulier d'extrait NOG (N-Octyl Glucoside) de la souche N6flbA- (à titre d'exemple, la quantité d'extrait pourrait être de 3 pg/ml ou la quantité d'antigène pourrait être de 2 µg/ml),
- une gamme de témoins négatifs et positifs (le témoin positif étant mis en oeuvre avec des dilutions différentes) est utilisée et en parallèle, on teste des sérums de patients dilués au 1/100ème (volume déposé 100 µl),
- une étape d'incubation est ensuite réalisée par exemple pendant une heure à 37°C, étape suivie de plusieurs lavages successifs et d'une incubation ultérieure, par exemple pendant 1 heure à 37°C, avec un conjugué monoclonal (de type IgG humaine marquée à la peroxydase) utilisé à différentes dilutions (par exemple à une dilution de 1/32000 pour un antigène et à une dilution de 1/64000 pour un extrait bactérien), le volume déposé est de 100 µl,
- après l'incubation avec le conjugué monoclonal, différents lavages sont pratiqués (par exemple 4) et la révélation de la réaction enzymatique par « OPD + substrat » est faite pendant 30 minutes à l'obscurité. La réaction enzymatique est ensuite arrêtée par addition de H₂SO₄ puis lecture des densités optiques D0 à 492 nm/620 nm.

L'invention vise par ailleurs l'utilisation, pour l'obtention d'anticorps contre H. pylori, d'une souche bactérienne ou d'un extrait de cette souche bactérienne, définis dans la demande.

Selon un mode de réalisation particulier de l'invention, une composition immunogène pour l'obtention d'anticorps contre H. pylori est caractérisée en ce qu'elle comprend une séquence d'acides aminés de la protéine FlbA.

Entre également dans le cadre de la présente invention, l'utilisation, pour la préparation d'une composition vaccinante pour l'obtention d'anticorps protecteurs contre une infection par H. pylori, d'une souche bactérienne ou d'un extrait bactérien selon les définitions de la demande.

Une telle utilisation, pour préparer une composition vaccinante pour l'obtention d'anticorps contre une infection par H. pylori, pour mettre en oeuvre à titre de principe actif, des antigènes de type uréase, en particulier des antigènes codés par les gènes ureA, ureB, ureC ou ureD et une protéine répondant à une séquence d'acides aminés définie plus haut.

L'invention vise aussi des sérums polyclonaux dirigés contre une souche de H. pylori obtenue à partir de la souche NCIMB 40512 selon la définition ci-dessus, ou contre la souche NCIMB 40747,

Ces anticorps sont obtenus par des techniques connues en soi et en particulier par immunisation d'un animal avec l'antigène choisi, suivie soit de la production et de la récupération des anticorps produits et de la sélection de ceux d'entre eux qui reconnaissent spécifiquement H pylori.

D'autres anticorps monoclonaux ou sérums polyclonaux selon l'invention sont dirigés contre une souche de H. pylori aflagellée telle que décrite dans les pages précédentes.

L'invention concerne en outre une composition pour la détection in vitro d'une infection par H. pylori dans un échantillon biologique, comprenant à titre de principe actif un sérum polyclonal obtenus contre une souche de H. pylori de phénotype aflagellé décrite dans la demande.
figure 1
1A : Carte de restriction du plasmide plLL570 et du mini transposon Tn3 contenant la cassette du gène de résistance à la kanamycine.
1B : Cartes de restriction linéaires des plasmides recombinants pSUS39 et pSUS207. Les chiffres indiqués correspondent à la taille des fragments de restriction exprimée en paires de bases. H : HindIII; Bg : BgIII. La présence d'un astérisque indique que le site de restriction a été modifié au cours du clonage et qu'il n'est plus reconnu par l'enzyme de restriction correspondant.

figure 2 : Séquence nucléotidique du gène flbA de H. pylori et séquence en acides aminés déduite, donnée en code à une lettre.

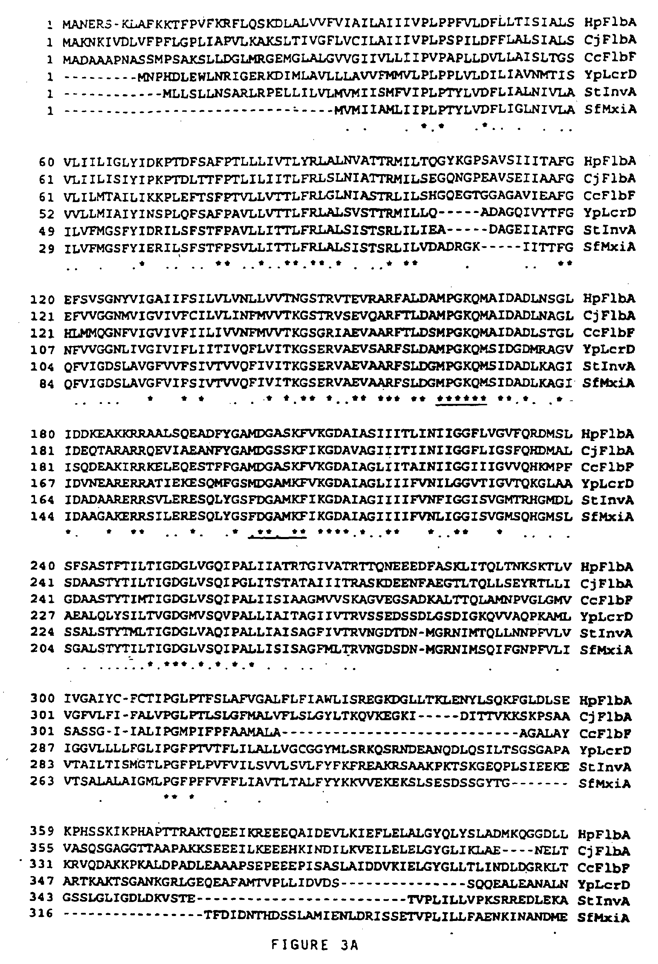

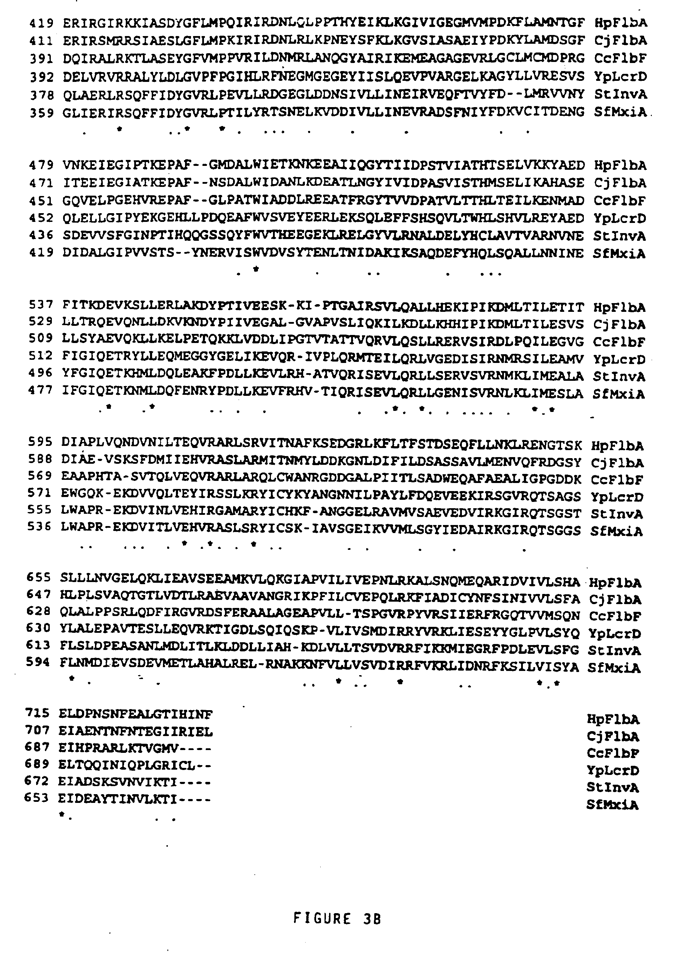

figure 3 : Alignement multiple de la protéine FlbA de H. pylori avec cinq autres membres de la famille LcrD/FlbF. CjFlbA : Campylobacter jejuni FlbA ; CcFlbF : Caulobacter crescentus FlbF ; YpLcrD : Yersinia pestis LcrD ; StInvA : Salmonella typhimurium InvA ; SfMxiA : Shigella flexneri MxiA. Les astérisques indiquent la position d'acides aminés conservés dans tous les homologues de la famille LcrD/FlbF ; les points indiquent la position d'acides aminés conservés dans au moins 5 des 6 protéines homologues ; les séquences en acides aminés conservés utilisées pour la synthèse des oligonucléotides dégénérés (OLFlbA-1 et OLFlbA-2) sont soulignées. On remarquera en particulier le degré de conservation du domaine N-terminal de ces protéines homologues qui contraste avec le degré de variabilité du domaine hydrophile de la région C-terminale.
figure 4 : Représentation schématique de l'arbre phylogénique de six protéines appartenant à la famille LcrD/FlbF. Les protéines impliquées dans la régulation de l'expression de la mobilité, FlbA de H. pylori (HpFlbA), et de Campylobacter jejuni (Cj FlbA), et FlbF de Caulobacter crescentus (Cc FlbF) forment une branche distincte de celle des protéines impliquées dans la sécrétion de protéines de virulence (InvA, MxiA et LcrD de Salmonella, Shigella et Yersinia, respectivement). Les nombres indiqués représentent la distance évolutionnaire relative.
figure 5 : Représentation schématique de la stratégie suivie pour construire les mutants isogéniques de H. pylori souche N6, mutants dans lesquels le gène codant pour la protéine FlbA a été inactivé par insertion d'un gène codant pour la résistance à la kanamycine.
figure 6 : Analyse par immunobuvardage (Western blot) des protéines d'un mutant N6-flbA à l'aide de l'antisérum AK179 (3) dirigé spécifiquement contre les flagelles purifiés de H pylori : 1 : mutant N6-flbA : 2 : double mutant flaA/flaB ; 3 : mutant flaB (8) ; 4 : mutant flaA (8) ; 5 : souche sauvage N6.

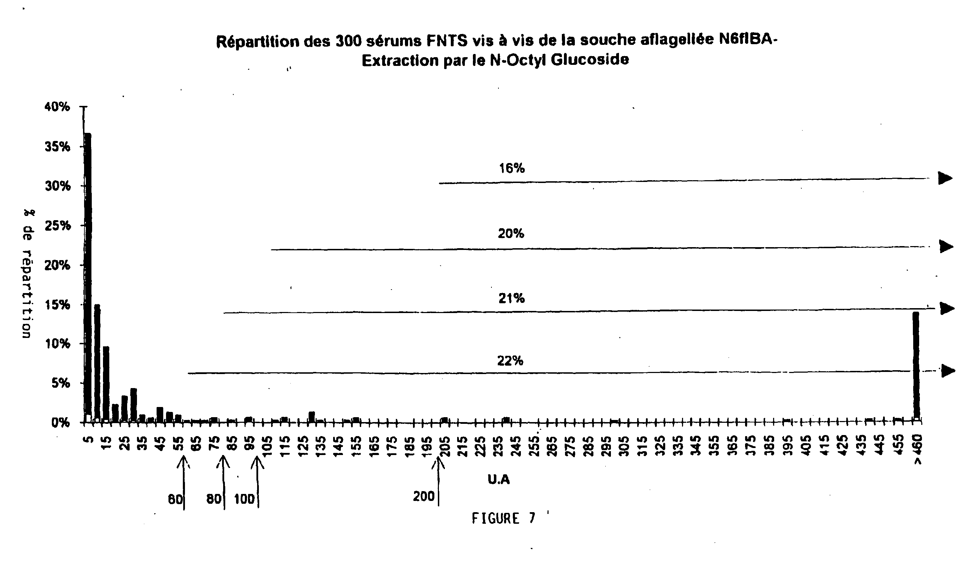

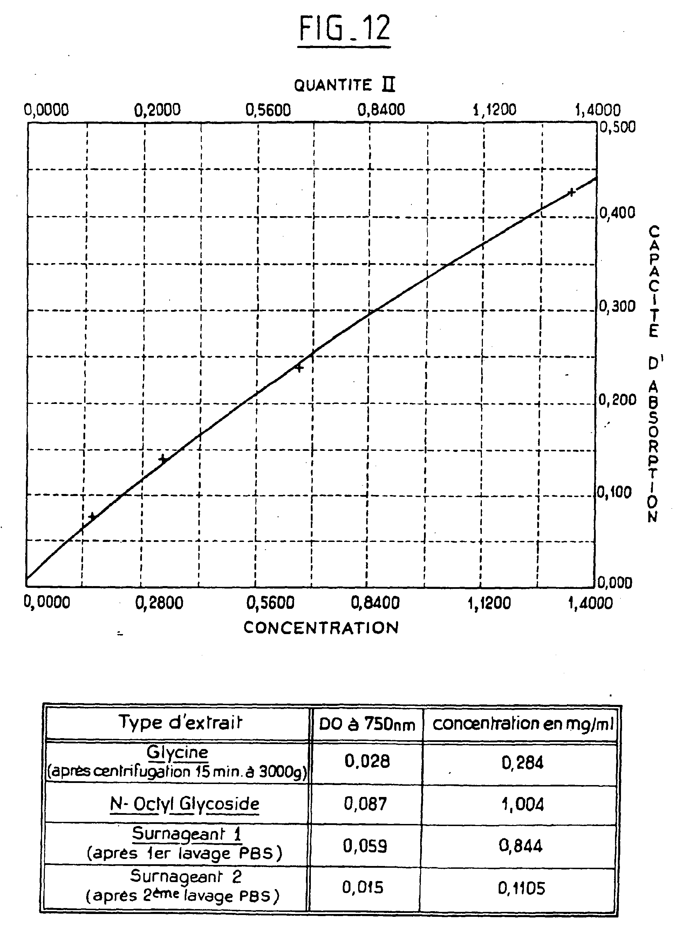

figures 7 à 11 : Résultats comparatifs de la sérologie réalisée sur H. pylori. figures 12 et 13 : Extractions à partir de la souche aflagellée N6flbA- : les extractions ont été réalisées avec de la glycine, du PBS, ou du NOG.
Figure 12 : Les courbes ont été réalisées à partir des données suivantes :

| | | NET ABS | CALC | | COEFFS: | | |
|---|---|---|---|---|---|---|---|
| STD# | CONC | 750.0 | CONC | DIFF | P2=2.0324 | P1=2.2753 | PO=-0 |
| 1 | 0.0000 | 0.0020 | -0.008 | 0.0080 | | | |
| 2 | 0.1660 | 0.0760 | 0.1721 | -0.006 | | | |
| 3 | 0.3300 | 0.1400 | 0.3459 | -0.016 | | | |
| 4 | 0.6650 | 0.2390 | 0.6474 | 0.0176 | | | |
| 5 | 1.3300 | 0.4280 | 1.3336 | -0.004 | | | |
| | | | | | MOYENNE: -1.0356E-07 | | |
| | | | | | S.D.: 0.0130 | | |

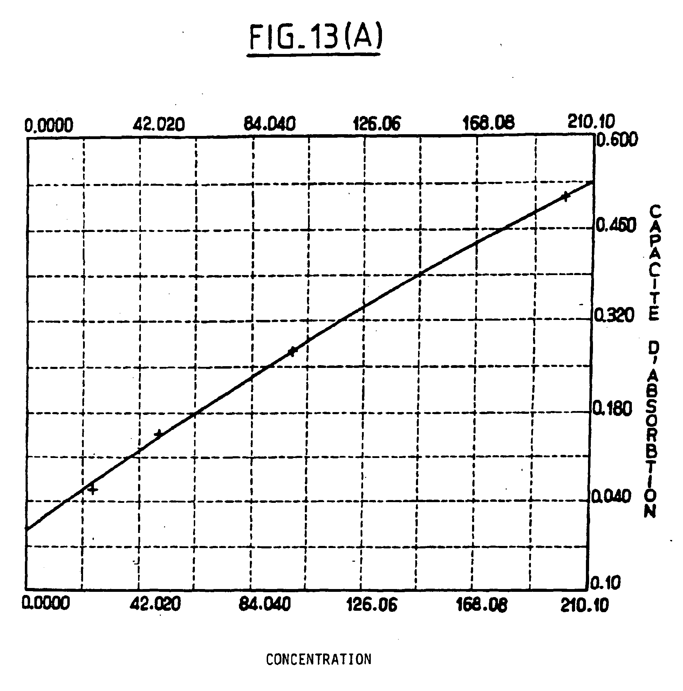

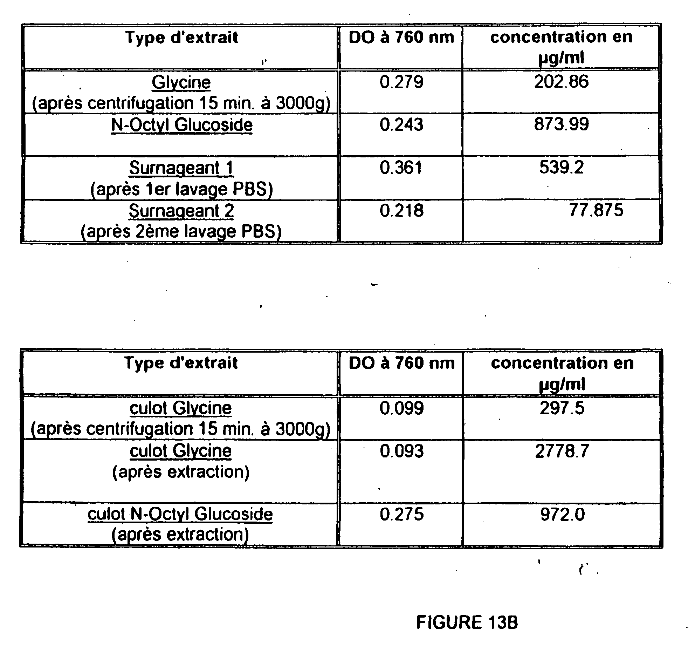

Figure 13: Dosages protéiques en minométhode (BIOD RAD) Glycine : dilution au 1/2 ; glucoside : dilution au 1/10 ; surnageant 1 : dilution au 1/4 ; surnageant 2 : non dilué.

Les courbes ont été réalisées à partir des données suivantes :

| | | NET ABS | CALC | | COEFFS: | | |
|---|---|---|---|---|---|---|---|
| STD# | CONC | 750.0 | CONC | DIFF | P2=144.63 | P1=314.31 | PO=2.4815 |
| 1 | 0.0000 | -0.003 | 1.5398 | -1.540 | | | |
| 2. | 25.000 | 0.0600 | 21.861 | 3.1392 | | | |
| 3 | 50.000 | 0.1470 | 51.810 | -1.810 | | | |
| 4 | 100.00 | 0.2750 | 99.855 | 0.1454 | | | |
| 5 | 200.00 | 0.5090 | 199.94 | 0.0636 | | | |

### EXEMPLES

### l'Identification du gène flbA et préparation de souches aflagellées

Parmi les protéines connues pour jouer un rôle dans la régulation de l'expression de la mobilité chez les bactéries, les protéines appartenant à la famille LcrD/FlbF récemment identifiée, et qui inclut la protéine LcrD des bactéries du genre Yersinia (6), la protéine InvA de Salmonella (2), MxiA de Shigella (1), FlbF de Caulobacter crescentus (7) et FlbA de Campylobacter jejuni (4) sont des protéines intéressantes. Les protéines LcrD, InvA et Mxia sont impliquées dans la régulation et/ou la sécrétion de protéines associées à la virulence des bactéries qui les expriment, alors que les protéines FlbF de Caulobacter crescentus et FlbA de Campylobacter jejuni sont impliquées dans la régulation de la biosynthèse des flagelles et donc de la mobilité. Les homologues connus à ce jour de la famille LcrD/FlbF ont des domaines très conservés surtout dans la partie N-terminale de ces protéines, et deux de ces régions conservées (MPGKQM, acides aminés 151 à 156 de la protéine LcrD de Yersinia) et MDGAMKF (acides aminés 189 à 195 de LcrD) ont ainsi pu être utilisées pour la définition de deux oligonucléotides dégénérés (OLFlbA-1 et OLFlbA-2, tableau 1) qui ont été synthétisés et ont servi d'amorces nucléotidiques dans des expériences d'amplification génique réalisées à partir de l'ADN chromosomique d'Helicobacter pylori. Un fragment de 130 paires de base (pb) a ainsi pu être amplifié et la détermination de sa séquence nucléotidique a montré que ce fragment codait pour un segment d'une protéine très homologue aux protéines de la famille LcrD/FlbF. Ce fragment amplifié a alors été marqué radioactivement et utilisé comme sonde pour cribler une banque cosmidique de H. pylori.

Ce fragment correspond à la séquence comprise entre les nucléotides 575 et 707 de la séquence représentée à la figure 2.

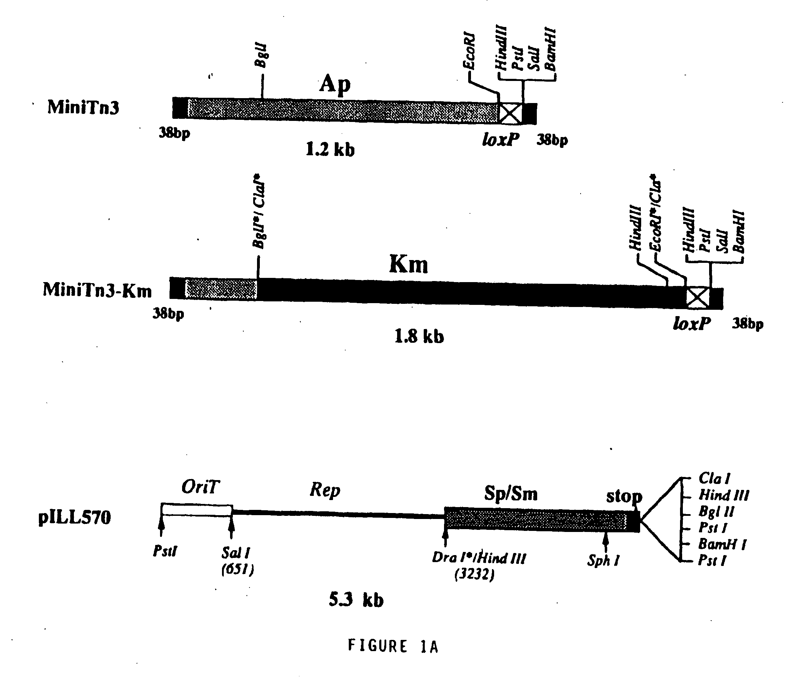

Un des cosmides de la banque génomique a été identifié comme codant pour l'homologue LcrD/FlbF de H. pylori, et a ensuite été soumis à une digestion partielle par Sau3A de façon a réaliser une mini banque (200 sous-clones) du cosmide dans le vecteur plLL570 contenant des fragments insérés ayant une taille comprise entre 2 et 5 (kilobases). Le vecteur plLL570 a été décrit dans la publication de Labigne A. et al (Institut Pasteur/Elsevier Paris 1992. Res. Microbiol. 1992, 143, 15-26). Sa carte de restriction est donnée à la figure 1A. Ces 200 clones ont alors été hybridés à la sonde de 130 pb et les clones hébergeant les plasmides pSUS39 et PSUS207 ont donné une hybridation positive. La carte de restriction linéaire de ces deux plasmides recombinants est représentée dans la figure 1B et montre que les deux inserts de ces clones ont des séquences chevauchantes. La détermination de la séquence nucléotidique de ces deux inserts a révélé qu'aucun des deux ne contenait le gène flbA dans son entièreté. Le gène flbA de H. pylori ainsi désigné du fait de son homologie avec le gène flbA de Campylobacter jejuni correspond à une phase ouverte de lecture de 2196 nucléotides et code pour une protéine de masse moléculaire calculée de 80,1 kilodaltons. La séquence nucléotidique de flbA et la séquence en acide aminés de FlbA sont données dans la figure 2. Il n'a pas été détecté en amont et en aval de la phase ouverte de lecture de séquences de consensus caractéristique de séquences promoteurs ou terminateurs.

La protéine FlbA présente des similitudes avec les protéines FlbA de Campylobacter jejuni et FlbF de Caulobacter crescentus, toutes deux impliquées dans la mobilité (51,7 % et 40,4 % d'identité, respectivement) alors que ces pourcentages sont moins élevés avec les membres de la famille protéique LcrD/FlbF qui ne sont pas impliqués dans la mobilité : 32,8 % d'identité avec LcrD de Yersinia, 30,5 % avec MxiA de Shigella et 29,3 % avec InvA de Salmonella. Un alignement multiple de la séquence en acides aminés de ces protéines avec celle de FlbA de H. pylori est donné dans la figure 3. Les régions les plus conservées des homologues de la famille LcrD/FlbF se trouvent dans la partie N-terminale des protéines.

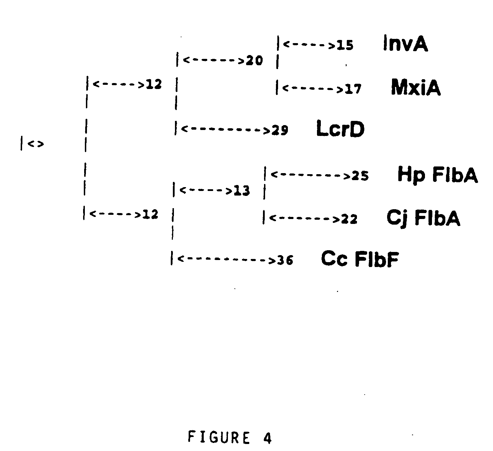

L'évolution phylogénétique des protéines impliquées dans la mobilité (FlbA et FlbF) et celle des protéines impliquées dans la régulation de l'expression et/ou la sécrétion de protéines associées à la virulence est représentée schématiquement par un arbre phylogénique (figure 4). Deux branches distinctes sont visibles ; FlbA de H. pylori appartenant sans ambiguité à la branche correspondant aux protéines de régulation impliquées dans la biosynthèse des flagelles.

### Construction et caractérisation de mutants isogéniques de H. pylori déficients dans la synthèse de la protéine FlbA.

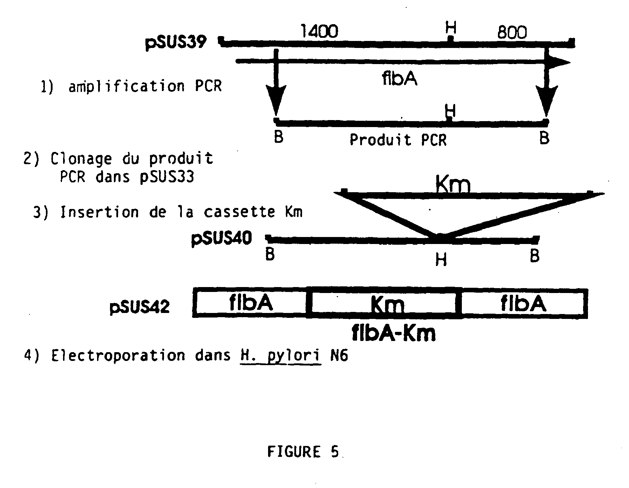

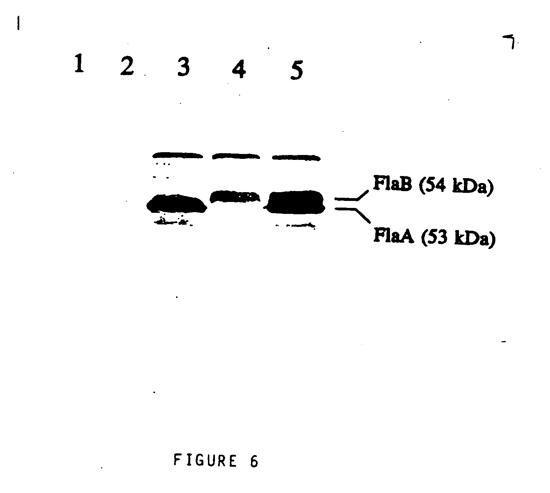

Un fragment de 1600 paires de bases a été amplifié à partir du plasmide pSUS39 à l'aide des oligonucléotides OLFlbA-7 et OLFlbA-8 (Tableau 1), contenant chacun à leur extrémité 5' des sites de restriction BamHI. Ce fragment amplifié contient dans sa région centrale un site unique de restriction par l'endonucléase HindIII et a été cloné dans le vecteur pSUS33, un dérivé du plasmide pUC19 dans lequel le site situé dans le site de polyclonage HindIII a été délété. Pour obtenir pSUS33, le plasmide pUC19 a été restreint par HindIII ; les extrémités collantes résultant de cette restriction ont été traitées avec l'enzyme de Klenow et l'ADN T4 polymérase de façon à produire des extrémités franches ; le fragment résultant a été religaturé avec l'ADN T4 ligase et introduit dans E. coli DH5x pour produire pSUS33. Le plasmide recombinant résultant de l'intégration du fragment de 1600 paires de base dans pSUS33 a été désigné pSUS42 ; il a été linéarisé par HindIII, ses extrémités ont été transformées en extrémité à bouts francs et la cassette smal - kanamycine provenant du plasmide plLL600 (Labigne A. et al, 1988, J. Bact., 170, 1704-1708) a été clonée dans ce site unique donnant naissance au plasmide pSUS42. Le plasmide pSUS42 a ensuite été introduit par électroporation dans la souche "N6" de H. pylori. L'électroporation a été réalisée en suivant la technique décrite par Ferrero R.L. et al (Journal of Bacteriology, July 1992, p. 4212-4217, vol. 174, n° 13). Les transformants obtenus après sélection sur un milieu sélectif contenant de la kanamycine (25 µg/ml) ont ensuite été caractérisés génotypiquement et phénotypiquement. La figure 5 montre un schéma de la procédure suivie pour réaliser la construction de mutants. La caractérisation génotypique de ces mutants par amplification génique et hybridation de type Southem a montré que le génome des transformants résistants à la kanamycine contenait le gène de résistance inséré au milieu du gène flbA, et donc qu'il y avait eu remplacement allélique par double crossing-over de la copie sauvage du gène flbA par la copie inactivée flbA-Km avec perte des séquences nucléotidiques du vecteur pSUS33 La caractérisation phénotypique des mutants flbA de H. pylori a montré qu'ils étaient non mobiles ; par ailleurs, leur analyse par microscopie électronique révélait qu'il y avait absence totale des éléments du flagelle, et absence de la gaine du flagelle. Les expériences d'immunobuvardages (Western blots) réalisées avec des anticorps dirigés contre les protéines des flagelles totales de H. pylori (Figure 6) montraient l'absence des deux bandes peptidiques correspondant aux sous-unités flagellaires FlaA et FlaB, ainsi qu'une bande correspondant à un polypeptide d'une masse apparente de 90 kilodaltons, protéine récemment identifiée comme la protéine du crochet (ou protéine d'ancrage) du flagelle par O'Toole et collaborateurs (5).

L'ensemble de ces résultats suggère que la protéine FlbA de H. pylori est essentielle pour la biosynthèse de toutes les structures flagellaires, et que l'activation du gène codant pour cette protéine, résulte en l'arrêt complet de la synthèse de toute structure entrant dans la constitution du flagelle et non pas dans l'interruption de l'exportation des constituants de ces structures.

**Tableau 1 : Oligonucléotides utilisés dans cette étude**

| Oligonucléotides | Position | Brin | Séquence nucléotidique |
|---|---|---|---|
| OLFlbA-1 | AS 151-156(LcrD) | + | ATGCCTCGAGGTCGAAAAGCAAGATG |
| OLFlbA-2 | AS 189-195 (LcrD) | - | GAAATCTTCATACTGGCAGCTCCAGTC |
| OLFlbA-7 | 515-534 | + | CGGGATCCGTGGTTACTAATGGTTCTAC |
| OLFlbA.8 | 2092-2111 | - | CGGGATCCTCATGGCCTCTTCAGAGACC |

### II Sérologie H. pylori

### - modèles étudiés

- 1) HspAmalE: protéine recombinante de 47,5 kD (HspA=13 kD) Sur la population dite population 1 de sérums documentés, une sensibilité de 41% et une spécificité de 96% avaient été obtenues.
- 2) N6flbA-: souche aflagellée d'Helicobacter pylori 3 extractions ont été réalisées : - N-Octyl Glucoside
- PBS
- Glycine
L'extraction au N-Octyl Glucoside (NOG) semble pour l'instant la meilleure.
- 3) -N6: souche sauvage correspondante
Une extraction au N-Octyl Glucoside a été réalisée.

Une deuxième population de sérums a été mise en oeuvre (population Il). Il s'agit d'une centaine de sérums bien documentés du point de vue clinique, endoscopique, histologique, bactériologique et anatomopathologique. C'est grâce à cette population II que les performances des différents modèles étudiés ont été appréciées. Cinq populations différentes ont été testées.

### - 5 Populations de sérums testées :

- 300 sérums tout venant (FNTS)
- 18 sérums positifs par la sérologie WHITTAKER (CBMS)
- 92 sérums bien documentés dits sérums de la population Il
- 87 sérums documentés du point de vue bactériologique et anatomopathologique dits sérums de la population I.
- 23 sérums pouvant présenter des réactions croisées :
   - 10 sérums anti-Legionella positifs
   - 10 sérums anti-Chlamydia positifs
   - 3 sérums anti-Campylobacter positifs

En parallèle, deux kits concurrents ont été testés, qui d'après les études bibliographiques sont performants.

### - 2 kits commerciaux testés :

- Cobas Core (ROCHE)
- Pylori Stat (WHITTAKER)

### - Résultats

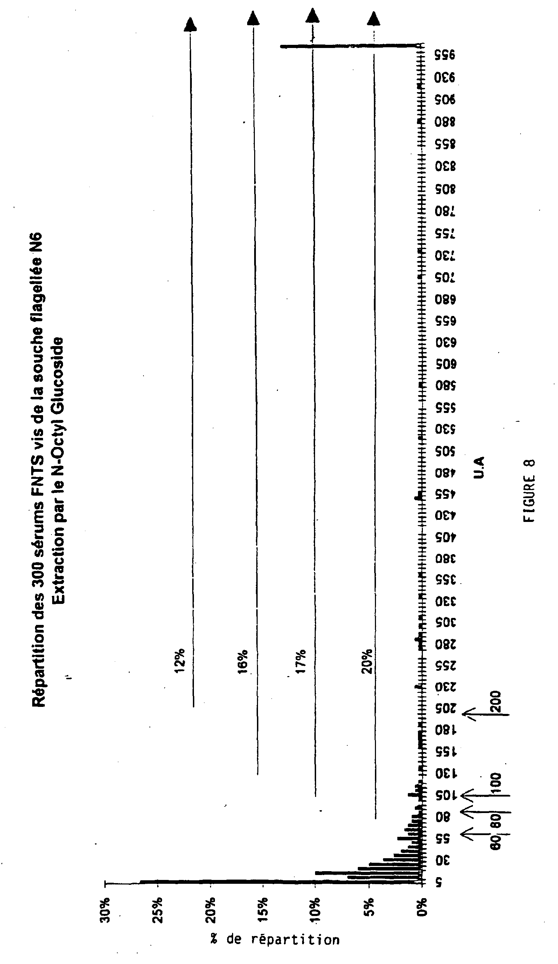

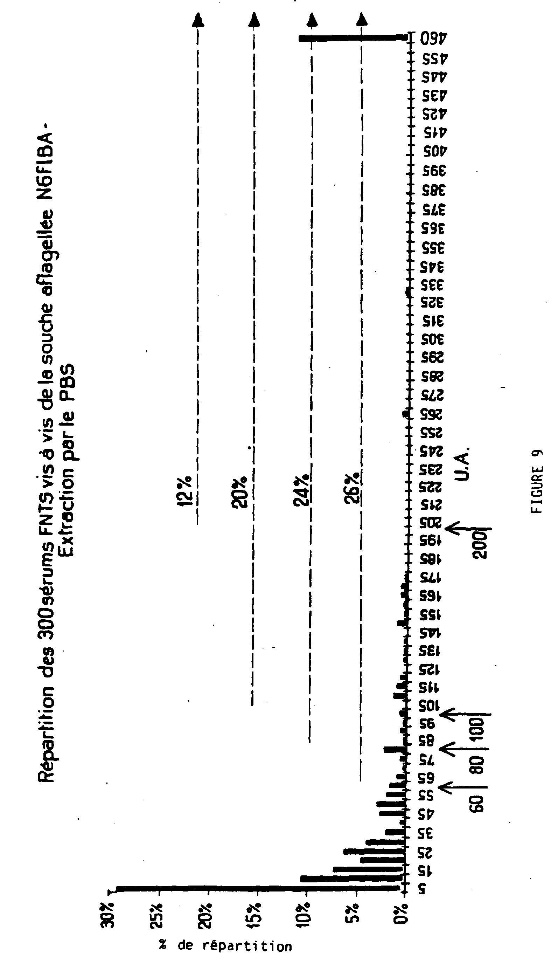

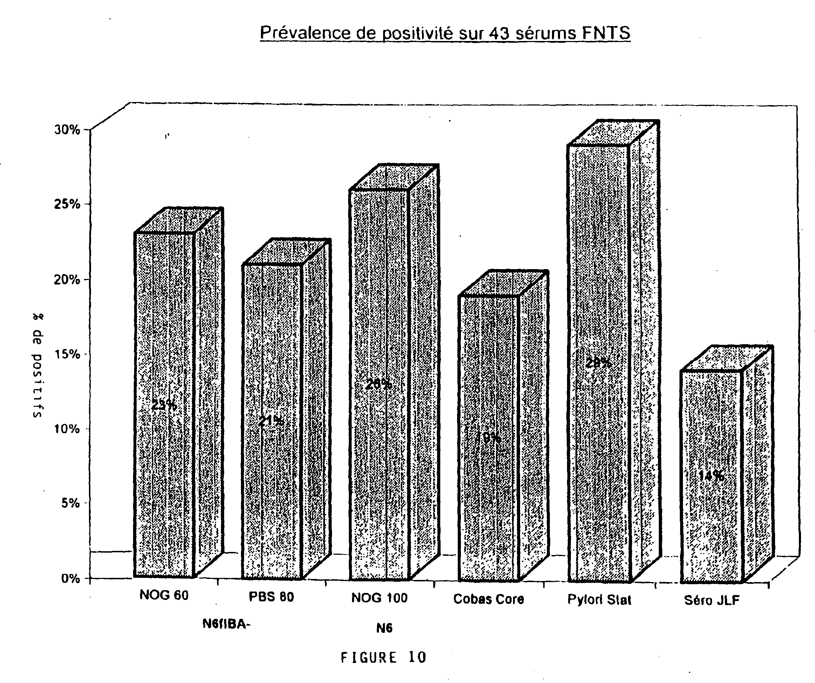

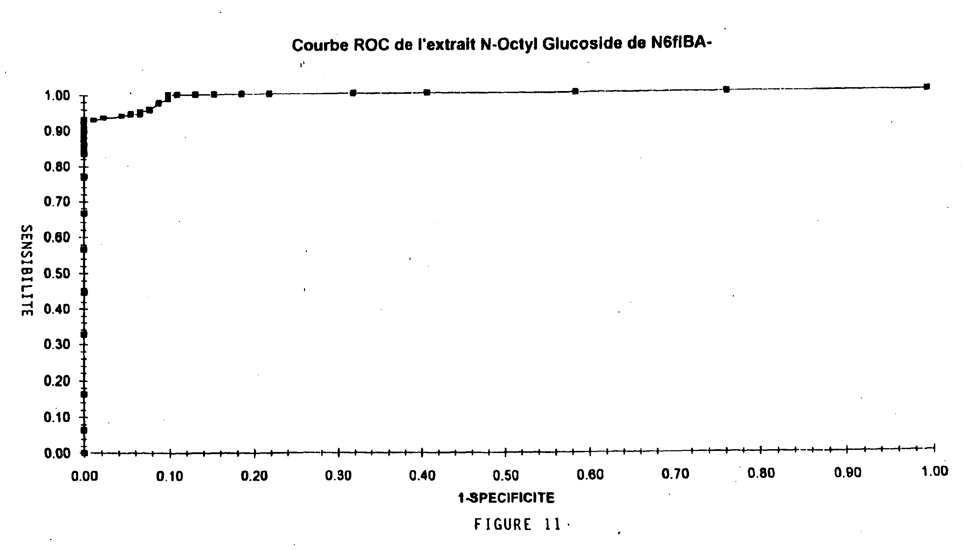

Les sérums tout venant (FNTS) (Figures 8 à 11, tableau 2)
- 300 sérums ont été passés dans les modèles suivants :
   - Hsp A malE
   - N6 flBA-
   - N6

Les études épidémiologiques donnent des séroprévalences, en France, entre 20 et 25%. La répartition de 300 sérums de donneurs de sang a été étudiée et on a calculé la prévalence de positivité pour différentes valeurs seuil, afin de valider la valeur seuil préalablement définie sur la sérothèque du CBMS (sérologie WHITTAKER).

Cette étude permet de comparer les différents tests avec une même séroprévatence.
- Les 43 premiers sérums ont également été passés dans les modèles suivants :
   - Cobas Core (ROCHE)
   - Pylori Stat (WHITTAKER)
   - sérologie dite JLF (test ELISA, basé sur un extrait
   aqueux de plusieurs souches bactériennes)
   Les résultats sont exprimés en unité arbitraire, et pour différentes valeurs seuil, un résultat positif est noté 1, un résultat négatif est noté 0.
   En comparant ces 43 sérums vis à vis des différents tests, on peut constater que :
   - la souche aflagellée N6flbA- et le test Cobas Core (Roche) ont des séroprévalences comparables de l'ordre de 20%.
   - HspA a une séroprévalence très faible (7%) ce qui laisse supposer un manque de sensibilité, compte tenu des résultats ultérieurs.
   - la sérologie JLF parait très spécifique puisque la séroprévalence n'est que de 14%, en tenant compte des résultats ultérieurs.
- le test Pylori Stat (Whittaker) donne une séroprévalence élevée (29%) qui montrerait un manque de spécificité, ou une valeur seuil trop basse.

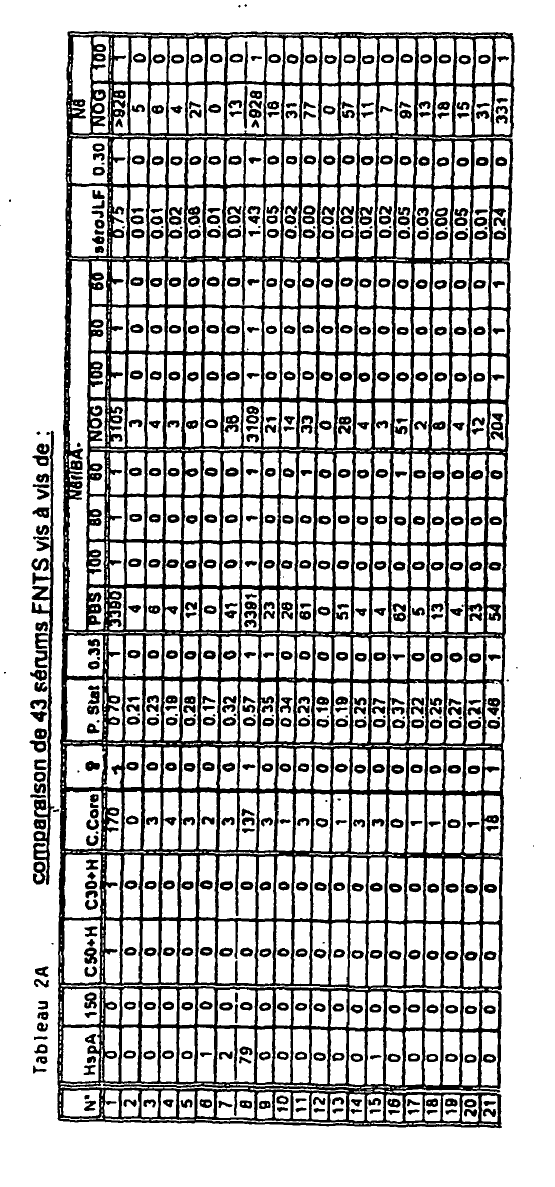

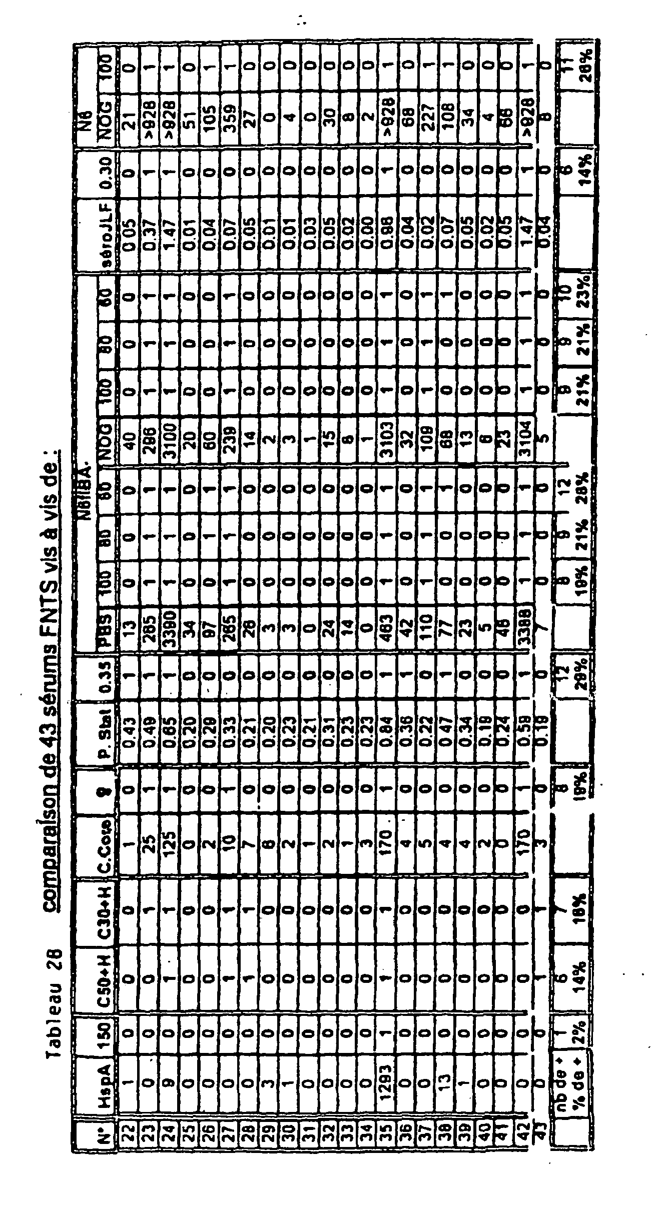

Les sérums positifs en sérologie WHITTAKER (CBMS) (tableau 3)
Trois sérums se sont révélés positifs uniquement avec le test Pylori Stat (Whittaker). Ils n'ont été confirmés par aucun autre test.
On peut supposer qu'il s'agit là d'un manque de spécificité de ce test. Si l'on prend comme référence le test Cobas Core (Roche) qui est l'un des meilleurs actuellement sur le marché, nous pouvons comparer nos différents modèles par rapport à Cobas Core.
- La souche aflagellée N6flbA- est parfaitement corrélée à Cobas Core.
- Les 3 sérums négatifs Cobas Core le sont également avec N6flbA-
- Les 15 sérums positifs Cobas Core le sont aussi avec N6flbA-.
- La souche sauvage N6 montre les mêmes performances que la souche aflagellée.
- HspA manque aussi de sensibilité car 9 sérums positifs Cobas Core sont négatifs avec HspA.
Les 3 sérums négatifs Cobas Core le sont aussi avec HspA.

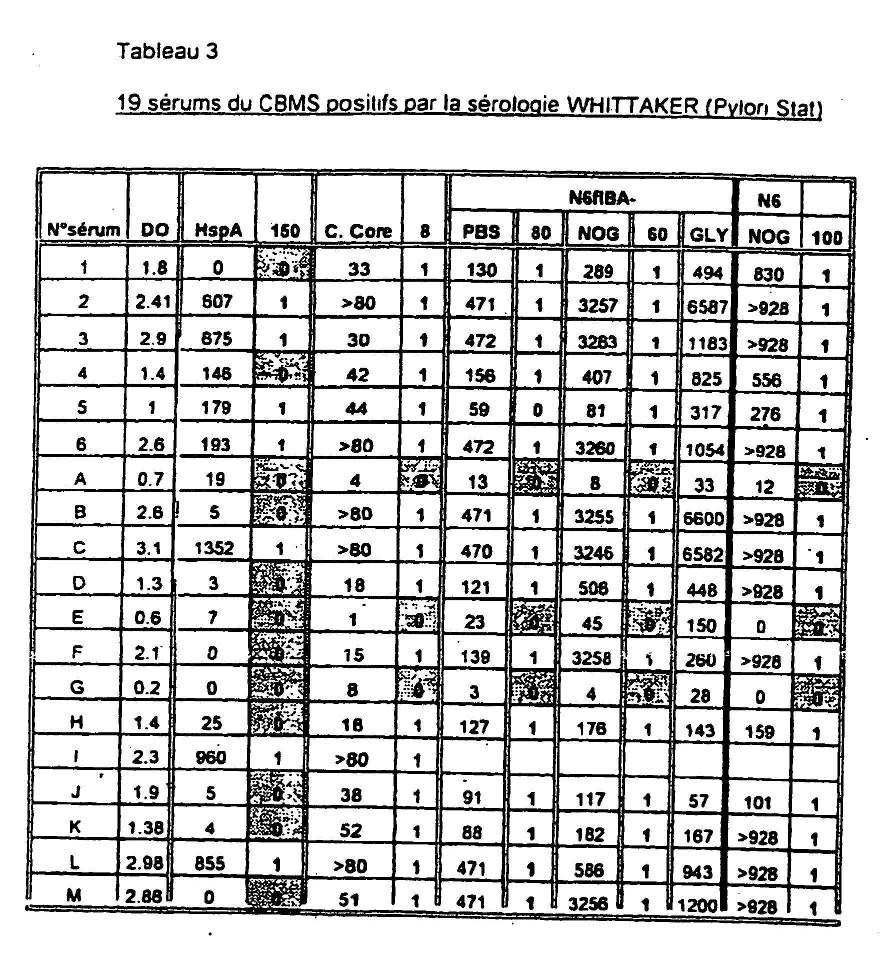

### Les sérums de la population II

92 sérums ont été sélectionnés et se répartissent en 3 groupes :
- -34 :: patients dyspeptiques
diagnostic d'ulcère (duodénal ou gastrique) à l'endoscopie et à l'histologie
présence d'Helicobacter pylori par culture et/ou anapath ; un test rapide à l'urée a également été réalisé.
Ce groupe sera noté Hp+/U+
- - 27 :: patients dyspeptiques
diagnostic différent d'ulcère (gastrite...) à l'endoscopie et à l'histologie
présence d'Helicobacter pylori par culture et/ou anapath ; un test rapide à l'urée a également été réalisé.
Ce groupe sera noté Hp+/U-
- - 31 :: patients dyspeptiques ou non
gastro-duodénum normal à l'endoscopie et à l'histologie
absence d'Helicobacter pylori par culture et anapath ; un test rapide à l'urée a également été réalisé.
Ce groupe sera noté Hp-

Pour chaque patient, les données cliniques, endoscopiques, histologiques, bactériologiques et anatomopathologiques sont indiquées.
Cette population bien documentée a permis de définir des critères de Sensibilité et de Spécificité.
- - HpA :: On note toujours un manque de sensibilité important comme constaté avec la population I.
La sensibilité est de 59% pour une spécificité de 100.
- - N6flbA- :: On vérifie une sensibilité de 100% pour l'extrait au N-Octyl Glucoside avec une spécificité de 90%.
La performance est comparable au test Cobas Core de Roche (sensibilité de 98% pour une spécificité de 94%).
- - N6 :: La souche sauvage est tout à fait comparable à la souche aflagellée sur la population II.
Aucun des 31 sérums négatifs n'est positif avec la souche sauvage ; aucune réaction croisée due au flagelle n'a été détectée sur cette population II.

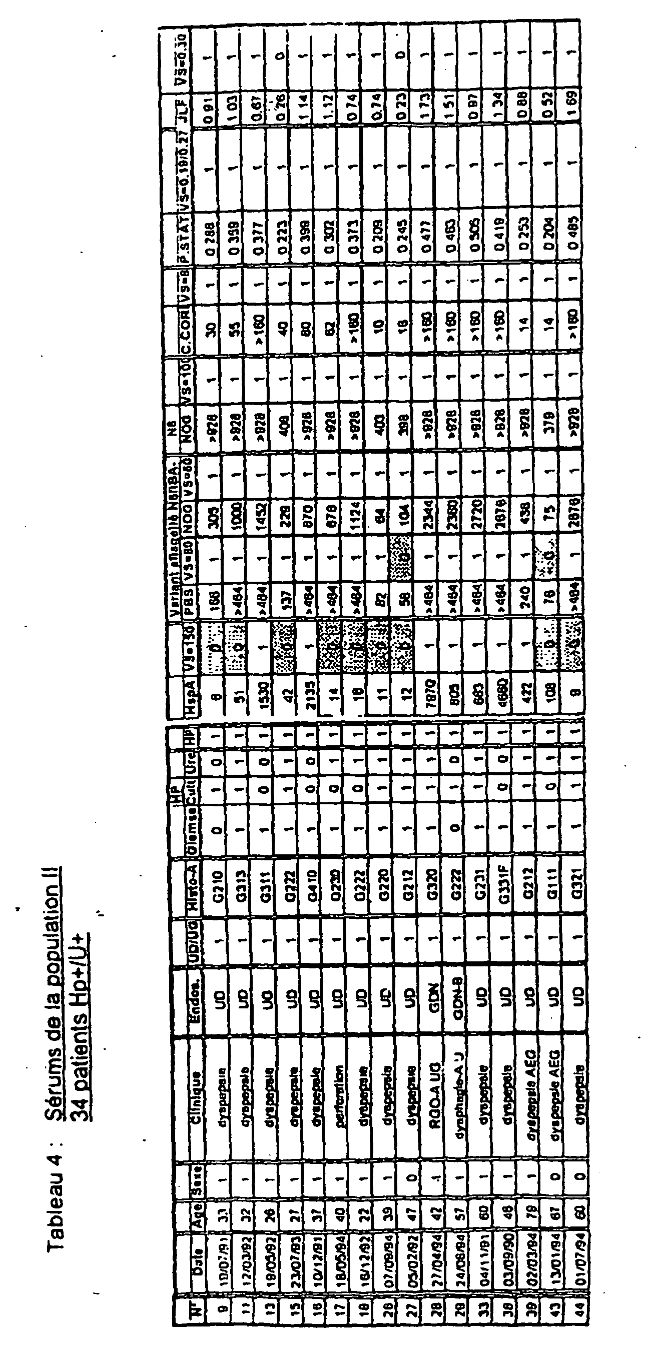

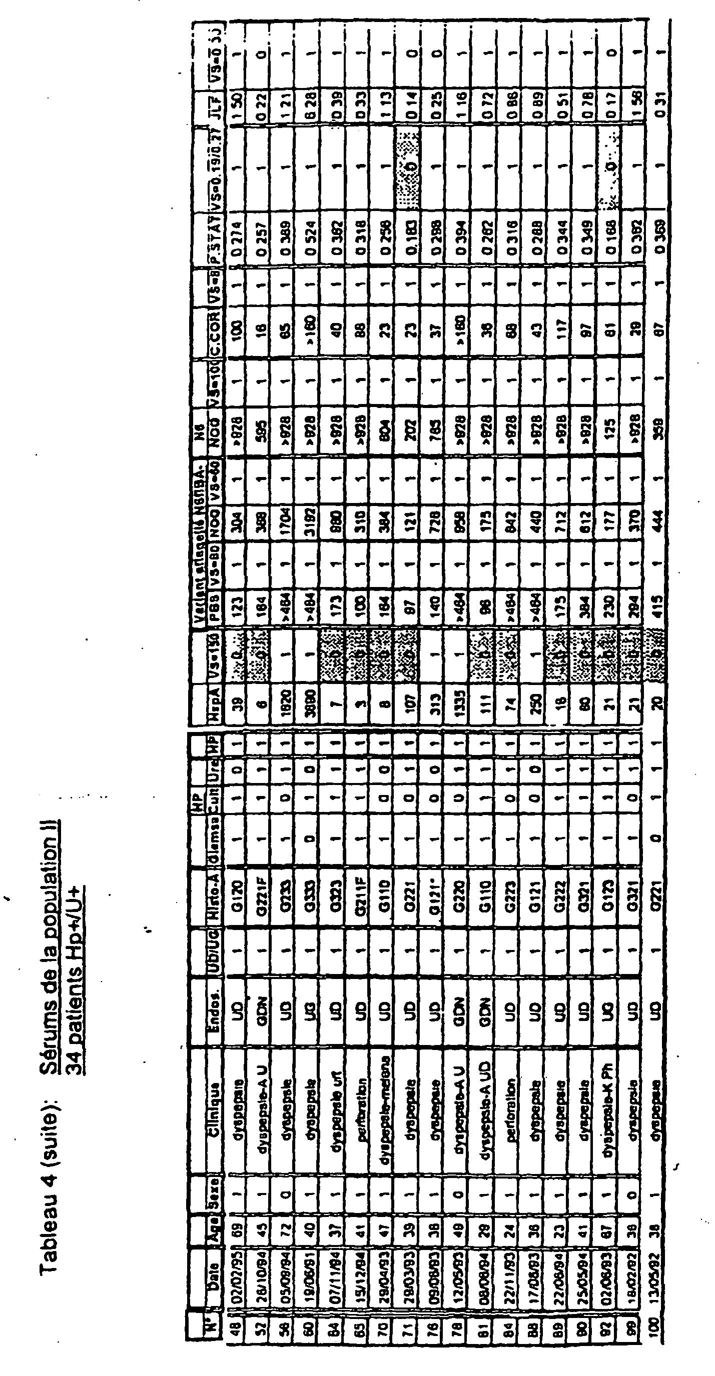

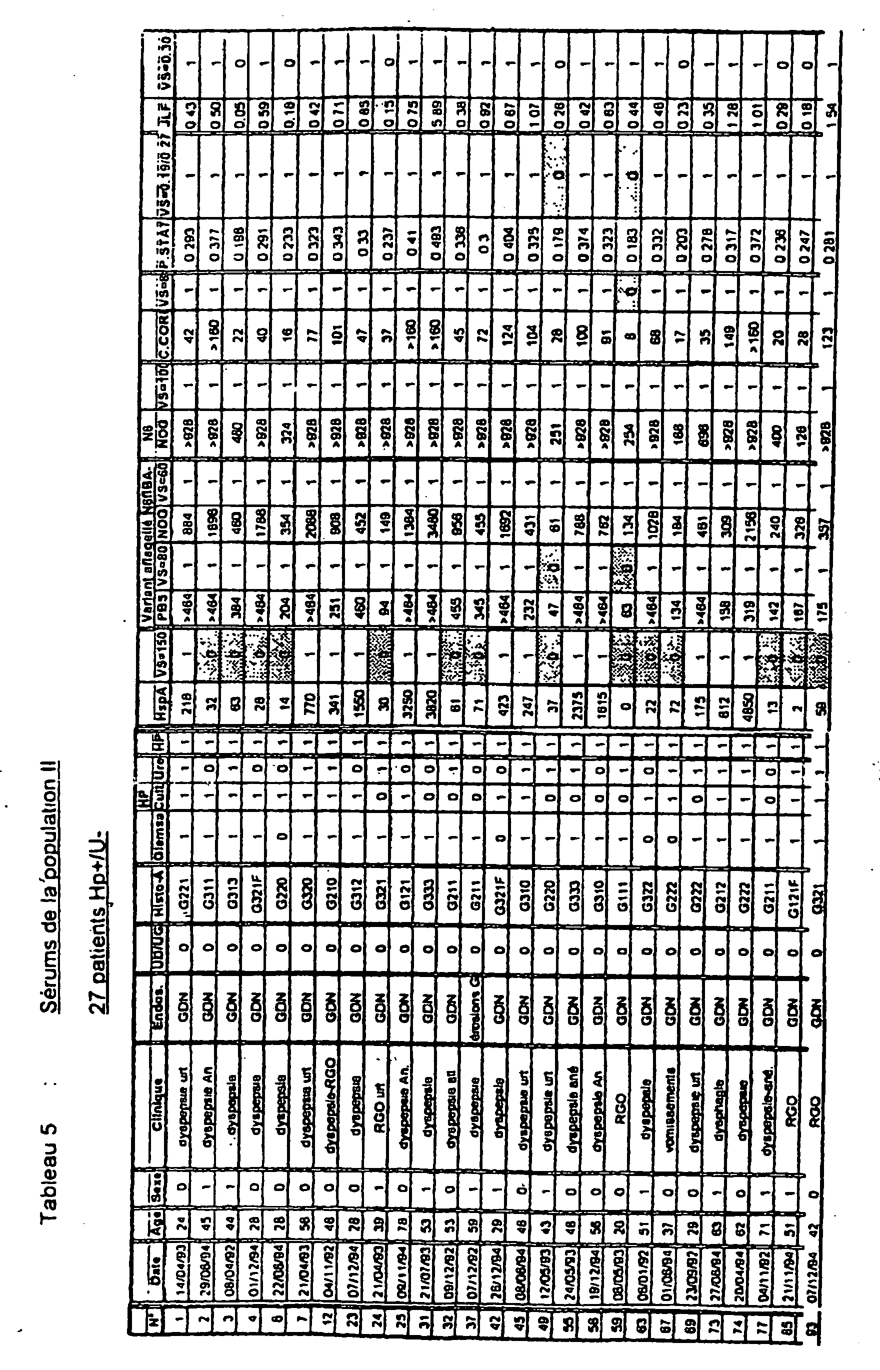

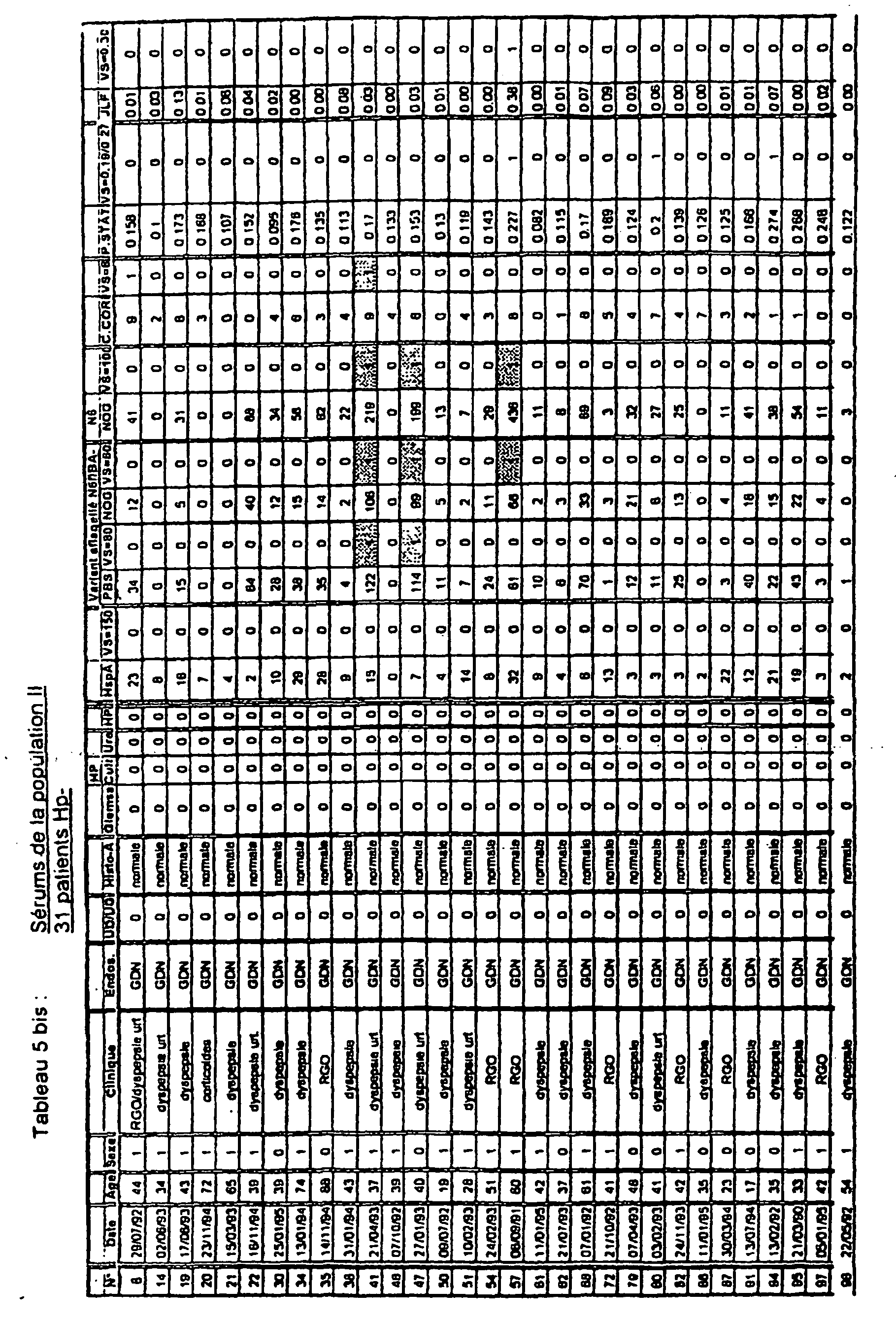

**Tableau 6 Sérums de population II Par rapport à la présence de Hp (culture et/ou anapath) et Ulcère**

| | | | | Sensibilité | Spécificité |
|---|---|---|---|---|---|
| Par rapport à Hp+ et UD/UG soit 34Hp+/U+ | HspA malE | | VS=100 | 44.1% (15/34) | *100% (31*/*31)* |
| | | | VS=50 | 52.9% (18/34) | *100% (31*/*31)* |
| | | | VS=20 | 64.7% (22134) | *73.8% (25*/*31)* |
| | N6flbA- | NOG | VS=100 | 94.1% (32/34) | *96.8% (30*/*31)* |
| | | | VS=80 | 94.1% (32/34) | *93.6% (29*/*31)* |
| | | | VS=60 | 100% (34/34) | *90.3% (28*/*31)* |
| | | PBS | VS=100 | 82.4% (28/34) | *(29*/*31) 93.6%* |
| | | | VS=80 | 94.1% (32/34) | *93.6% (29*/*31)* |
| | | | VS=60 | 97.1% (33/34) | *83.9% (26*/*31)* |
| | Séro JLF | | VS=0.30 | 82.4%(28/34) | *96.8% (30*/*31)* |
| | Pylori Stat | | | 94.1% (32/34) | *90.3% (28*/*31)* |
| | Cobas Core | | | 100% (34/34) | *93.6% (29*/*31)* |

**Tableau 7 : Sérums de population II Par rapport à la présence de Hp (culture et/ou anapath)**

| | | | | **Sensibilité** | **Spécificité** |
|---|---|---|---|---|---|
| Par rapport à Hp+ - 34 UD/UG - 27 GNU soit : 61 Hp+ 31 Hp- | HspA malE | | VS=100 | 45.9% (28/61) | *100% (31*/*31)* |
| | | | VS=50 | 59% (36/61 ) | *100% (31*/*31)* |
| | | | VS=20 | 80.7% (45/61) | *73.8% (25*/*31)* |
| | N6flbA- | NOG | VS=100 | 95.1% (58/61) | *96.8% (30*/*31)* |
| | | | VS=80 | 95.1% (58/61) | *93.6 % (29*/*31)* |
| | | | VS=60 | 100% (61/61) | *90.3% (28*/*31)* |
| | | PBS | VS=100 | 85.3% (52/61) | *93.6% (29*/*31)* |
| | | | VS=80 | 93.4% (57/61) | *93.6% (29*/*31)* |
| | | | VS=60 | 96.7% (59/61) | *83.9% (26*/*31)* |
| | Séro JLF | | VS=0.30 | 78.7%(48/61) | *96.8% (30*/*31)* |
| | pylori Stat | | | 93.4% (57/61) | *90.3 % (28*/*31)* |
| | Cobas Core | | | 93.3% (60/61)* | *93.6% (29*/*31)* |

| | | | | | |
|---|---|---|---|---|---|
| * sérum = VS | | | | | |

**Tableau 8 : Sérums de population II Par rapport à la présence de Hp (culture et/ou anapath) et l'absence d'Ulcère**

| | | | | **Sensibilité** | **Spécificité** |
|---|---|---|---|---|---|
| Par rapport à Hp+ et GNU soit : 27 Hp+/U- | HspA malE | | VS=100 | 48.2% (13/27) | *100% (31*/*31)* |
| | | | VS=50 | 66.7% (18/27) | *100% (31*/*31)* |
| | | | VS=20 | 85.2% (23/27) | *73.8% (25*/*31)* |
| | N6flbA- | NOG | VS=100 | 96.3% (26/27) | *96.8%* (30/31) |
| | | | VS=80 | 93.6% (26/27) | *93.6% (29*/*31)* |
| | | | VS=60 | 100% (27/27) | *90.3% (28*/*31)* |
| | | PBS | VS=100 | 88.9% (24/27) | *93.6% (29*/*31)* |
| | | | VS=80 | 92.6% (25/27) | *93.6% (29*/*31)* |
| | | | VS=60 | 96.3% (26/27) | *83.9% (26*/*31)* |
| | Séro JLF | | VS=0.30 | 74.1% (20/27) | *96.8% (30*/*31)* |
| | Pylori Stat | | | 92.6% (25/27) | *90.3% (28*/*31)* |
| | Cobas Core | | | 96.3%(26/27) | *93.6% (29*/*31)* |

### La place de la sérologie

La sérologie est placée à 2 niveaux :
- Sérologie très sensible : afin de dépister le portage de la bactérie chez les sujets jeunes se plaignant de douleurs épigastriques. Si la sérologie s'avère négative, le sujet ne subira pas d'endoscopie ni de biopsie, et une autre cause à ses douleurs sera recherchée.
- Sérologie spécifique de risque : il s'agit là de mettre en évidence le risque de faire une infection grave à Helicobacter pylori c'est à dire un ulcère, un cancer ou un lymphome gastrique (lymphome du MALT).
- soit en utilisant une molécule spécifique du risque en question
- soit en utilisant un seuil spécifique de risque
(valeur seuil plus élevée chez les sujets à risque que chez les sujets non à risque).

Cette sérologie spécifique peut être utilisée pour faire un screening de la population générale, et ainsi dépister les cancers et les lymphomes, liés à Helicobacter pylori qui ne seraient pas dépistés par fautes de symptômes. (Seuls les sujets se plaignant de douleurs vont consulter un gastro-entérologue).

La réponse à la question de sensibilité est bonne.

**Tableau 9 : Moyenne et écart type des U.A. dans les 3 groupes de patients.**

| | | Hp- (n=31) | Hp+/U- (n=27) | Hp+/U+ (n=34) |
|---|---|---|---|---|
| Hsp A | moyenne | 10.61 | 775.72 | 770.32 |
| | écartype | 8.81 | 1312.56 | 1666.52 |
| N6flBA- (NOG) | moyenne | 17.16 | 895.50 | 944.85 |
| | écartype | 26.69 | 818.57 | 915.27 |

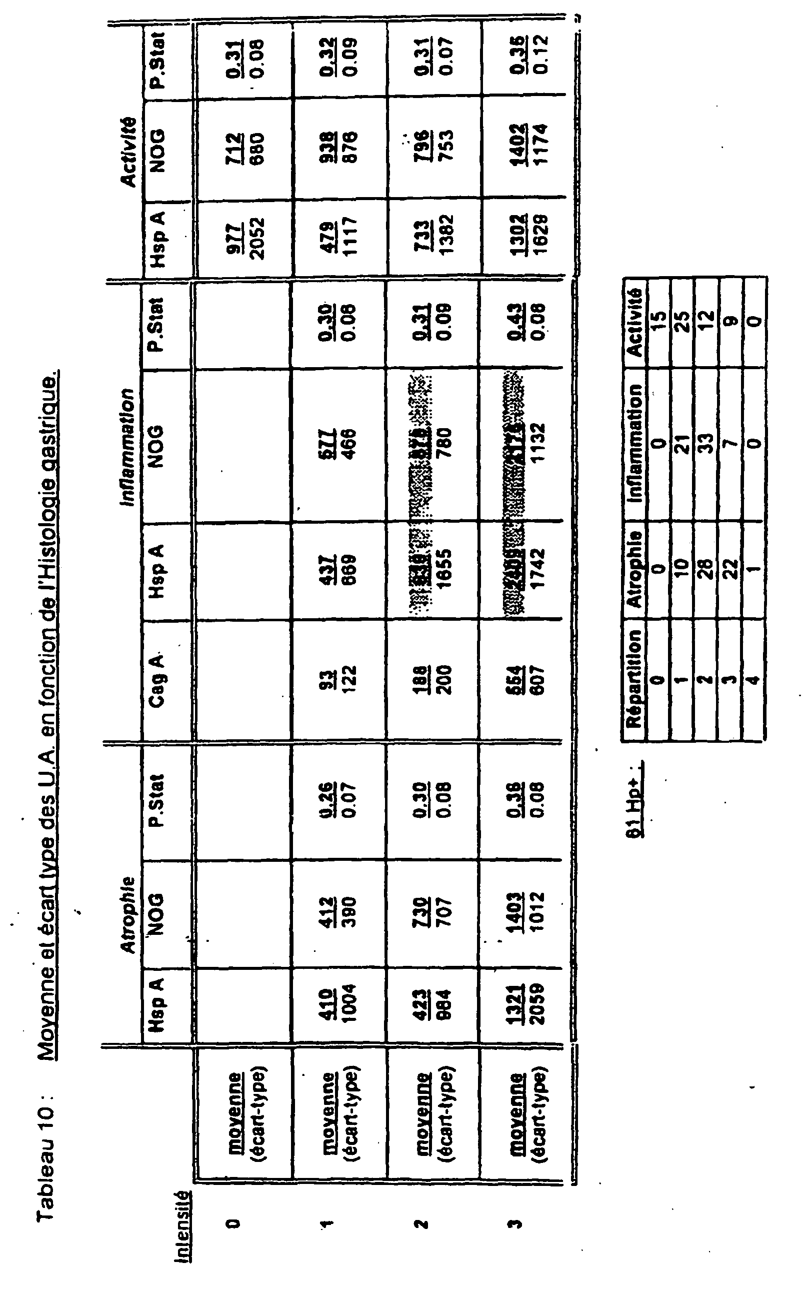

### Corrélation entre l'intensité de la gastrite et le taux en anticorps

La gastrite se définit par 3 paramètres :
- L'atrophie (représentée par le 1er chiffre après G) ; son intensité est notée de 1 à 4.
- L'inflammation globale correspond à l'infiltration de Polynucléaires neutrophiles et de Monocytes (représentée par le 2ème chiffre après le G). Son intensité est notée de 1 à 3.
- L'activité correspond au nombre de Polynucléaires neutrophiles (représentée par le 3ème chiffre après le G) ; son intensité est notée de 0 à 3. Certaines formes folliculaires sont notées F.
Normalement, on peut observer la corrélation suivante :
L'activité est très bien corrélée à Helicobacter pylori.
L'inflammation est bien corrélée à Helicobacter pylori.
On a donc calculé en fonction de ces 3 paramètres et de leur intensité, la moyenne des titres observés dans chaque groupe.

### Interprétation des résultats :

L'utilisation d'un test t permettra de mettre en évidence une différence significative ou non entre 2 moyennes observées avec un risque de 5%. L'hypothèse sur laquelle est fondée le test t est l'égalité des variances, mise en évidence par un test F (test de Fisher).
Certaines variances n'étant pas égales, on ne pourra donc pas comparer toutes les moyennes entre elles.
Les comparaisons des moyennes, lorsque cela est possible, ont pu mettre en évidence des différences significatives ou non dans les différents groupes.

### - Différence significative :

Dans le groupe « Inflammation », entre les moyennes des « 2 » et « 3 » de HspA et de NOG.

### - Différence non significative :

Au niveau de l'activité, on n'a pas mis en évidence de différence significative entre les différents niveaux d'intensité :

| | |
|---|---|
| - HspA : pas de différence significative entre les niveaux | 0 et 2 |
| | 0 et 3 |
| | 1 et 2 |
| | 1 et 3 |
| | 2 et 3 |
| - NOG : pas de différence significative entre les niveaux | 0 et 1 |
| | 0 et 2 |
| | 1 et 2 |
| | 1 et 3 |
| | 2 et 3. |

On peut néanmoins constater une tendance à l'augmentation des titres en fonction de l'intensité de la gastrite :
- au niveau de l'atrophie, les moyennes doublent lorsque l'on passe du niveau 1 à 2 et du niveau 2 à 3 pour HspA et pour l'extrait au NOG de la souche aflagellée.
- au niveau de l'inflammation, les moyennes doublent lorsque l'on passe du niveau 1 à 2.
Le nombre d'effectifs dans chaque groupe est relativement faible (toujours <30) pour conclure à des différences statistiquement significatives.

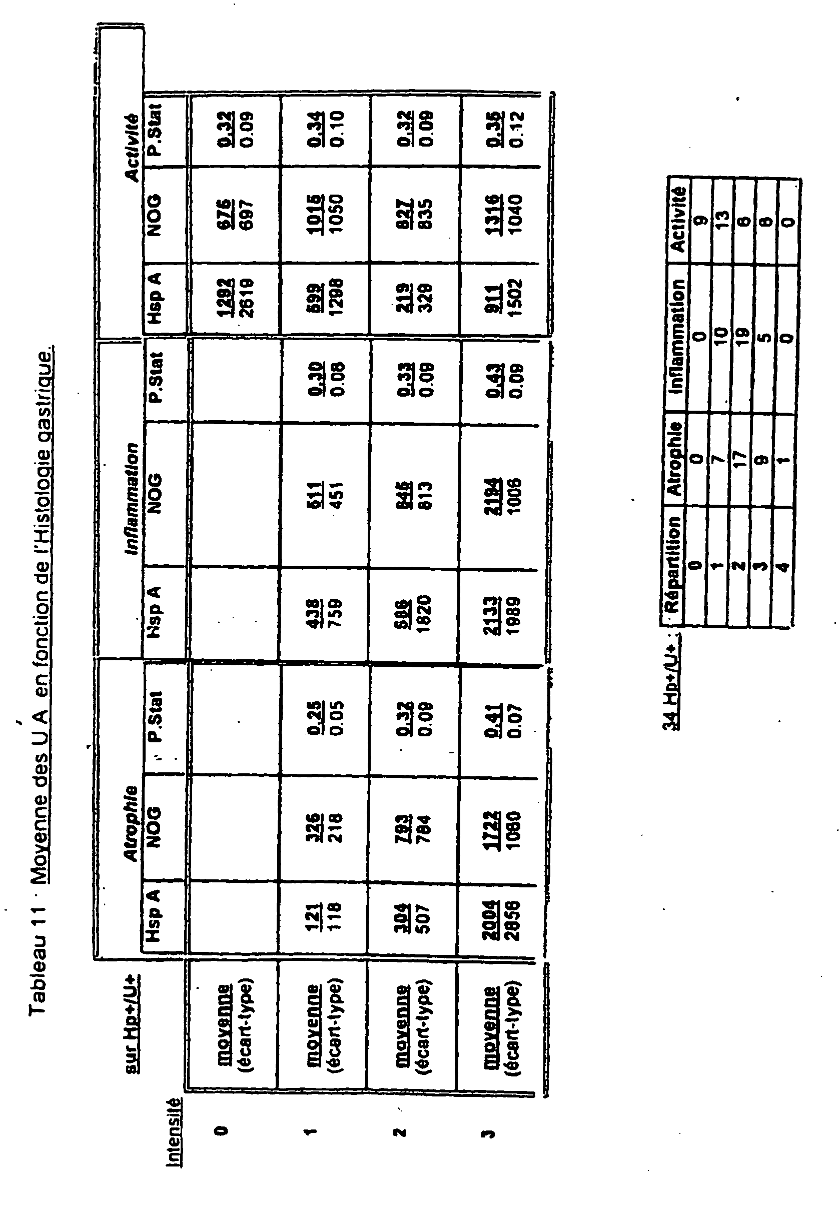

### Les sérums pouvant présenter des réactions croisées

2 types de sérums ont été utilisés.
20 sérums (10 anti-Legionella + et 10 anti-Chlamydia +) pouvant présenter des réactions croisées avec HspA, car ces 3 bactéries possèdent des « Heat shock proteins » très voisines les unes des autres.
3 sérums anti-Campylobacter positifs, afin de mettre en évidence des réactions croisées avec la souche flagellée N6, qui disparaîtraient avec la souche aflagellée N6flbA-. Il est très difficile de se procurer des sérums anti-Camylobacter positifs, c'est pourquoi il n'y a que 3 sérums.
HspA ne présente aucune réaction croisée, ni avec les 10 sérums anti-Legionella positifs, ni avec les 10 sérums anti-Chlamydia positifs.
Certains de ces sérums ont des titres positifs en anticorps anti-Helicobacter pylori, aussi bien avec la souche flagellée qu'avec la souche aflagellée, mais le contexte clinique de ces sérums n'est pas connu.

**Tableau 12 : Sérums pouvant présenter des réactions croisées**

| **Legionella +** | **Titre** | **N6** | **VS=100** | **N6flBA-** | **VS=60** | **HspA** | **VS=100** |
|---|---|---|---|---|---|---|---|
| A | P2 P3 =256 | 0 | 0 | 4 | 0 | 47 | 0 |
| B | P4 P5 =64 | >928 | 1 | 641 | 1 | 42 | 0 |
| C | P2 P3 =128 | 212 | 1 | 87 | 1 | 68 | 0 |
| D | P2 P3 =84 | 70 | 0 | 19 | 0 | 15 | 0 |
| E | P1=258/P2=512 | >928 | 1 | 239 | 1 | 258 | 1 |
| F | P2 P3 P4 P5 =128 | 322 | 1 | 121 | 1 | 41 | 0 |
| G | P1=512/P6=1024 | >928 | 1 | 193 | 1 | 121 | 1 |
| H | P4 P5 =64 | >928 | 1 | 479 | 1 | 18 | 0 |
| I | P2=128/P3=64 | 33 | 0 | 17 | 0 | 25 | 0 |
| J | P2=256/P3=128 | 16 | 0 | 8 | 0 | 32 | 0 |
| | | | | | | | |

| **Chlamydia +** | **Titre** | **N6** | **VS=100** | **N6flBA-** | **VS-60** | **HspA** | **VS=100** |
|---|---|---|---|---|---|---|---|
| A | 256 | 6 | 0 | 8 | 0 | 25 | 0 |
| B | 256 | 7 | 0 | 9 | 0 | 34 | 0 |
| C | 64 | 636 | 1 | 290 | 1 | 39 | 0 |
| D | 256 | 367 | 1 | 225 | 1 | 19 | 0 |
| E | 32 | >928 | 1 | 855 | 1 | 19 | 0 |
| F | 128 | >928 | 1 | 783 | 1 | 27 | 0 |
| G | 32 | 115 | 1 | 55 | 0 | 15 | 0 |
| H Twar | 16 | 19 | 0 | 10 | 0 | 14 | 0 |
| I | 32 | >928 | 1 | 592 | 1 | >928 | 1 |
| JTwar | 64 | 610 | 1 | 280 | 1 | 44 | 0 |
| | | | | | | | |

| **Campylobacter +** | | **N6** | **VS=100** | **N6flBA-** | **VS=60** | **HspA** | **VS=100** |
|---|---|---|---|---|---|---|---|
| A | | 35 | 0 | 28 | 0 | 17 | 0 |
| B | | 13 | 0 | 4 | 0 | 27 | 0 |
| C | | 50 | 0 | 68 | 1 | 89 | 0 |

### CONCLUSION

### HspA malE

Cette molécule ne peut toujours pas être utilisée seule car elle manque aussi de sensibilité, mais elle pourrait être intéressante si on l'associe à d'autres molécules.

Elle présente toutefois un risque de réactions croisées, du fait de la conservation importante de ces « Heat shock proteins » entre les différentes espèces bactériennes

### N6flbA-

Ce variant aflagellé paraît très intéressant, la sensibilité et la spécificité obtenues avec la population II de sérums montre une performance très intéresssante.

### N6

Pour l'instant, la souche flagellée semble intéressante. Cependant, les réactions croisées relatives au flagelle n'ont été que peu étudiées en raison de la difficulté à s procurer des sérums bien documentés en sérologie Campylobacter.

### Test JLF

Un test sérologique basé sur l'extraction aqueuse (PBS) de plusieurs souches d'Helicobacter pylori a été mis au point. Ce test semble très performant.

Un extrait NOG du variant aflagellé a été utilisé pour tester la population I de sérums.

87 sérums, documentés du point de vue bactériologique et anatomopathologique uniquement ont été testés avec l'extrait bactérien aflagellé.

Un sérum est positif si la culture est positive, ou si l'anapath, et le test rapide à l'urée sont positifs.

Un sérum est négatif si les 3 tests (culture, anapath, et test rapide à l'urée) sont négatifs.

On trouve une Sensibilité de 90.3% (28/31) pour une Spécificité de 71.4% (40/56).

Sur 16 sérums faux positifs avec un premier test, 9 sont positifs soit avec la sérologie JLF, soit avec le Western Blot JLF, soit avec les deux.

Sur les 3 sérums faux négatifs avec un premier test, les 3 sont négatifs, soit avec la sérologie JLF, soit avec le Western Blot JLF, et un sérum est négatif avec les deux systèmes.

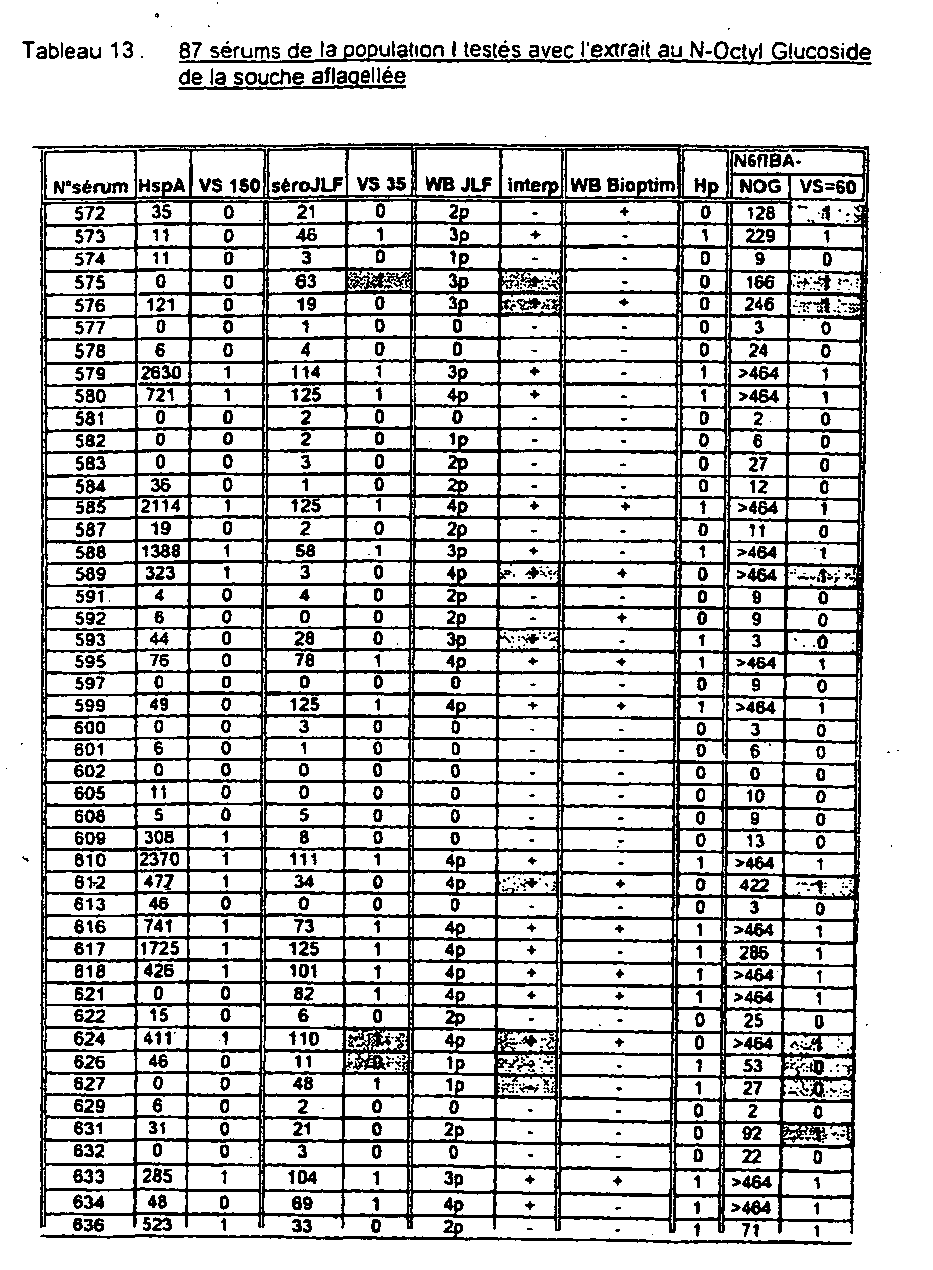

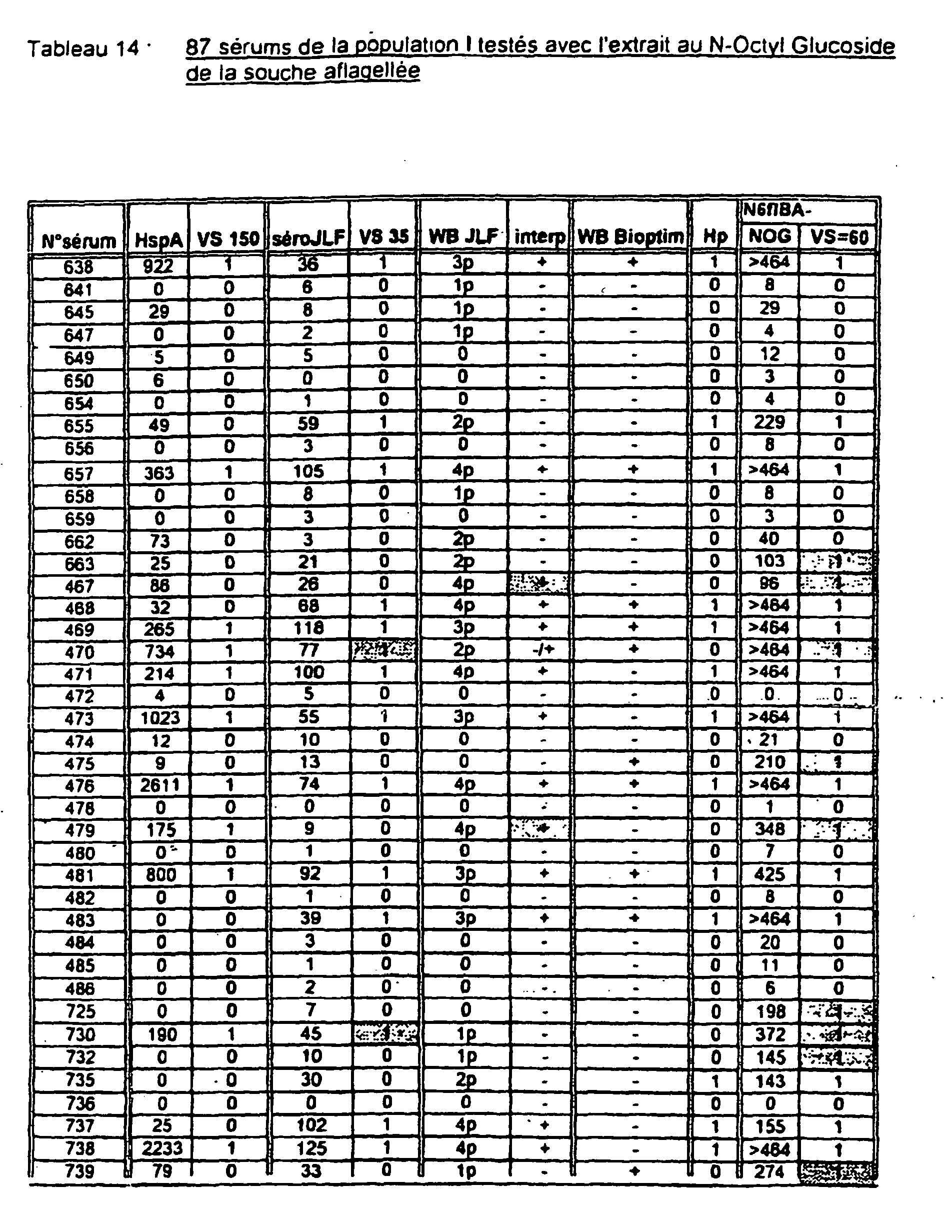

### TECHNIQUE

| | | |
|---|---|---|
| Plaques coatées à : | 2 µg/ml en Antigène Hsp A | |
| | 3 µg/ml en extrait NOG de NflbA et N6 | |
| Gamme: | 5 points de gamme | témoin négatif |
| | | témoin positif utilisé à 4 dilutions |
| | | |
| Sérums de patients: | dilution au 1/100 | |
| | volume déposé | 100µl |
| | | |
| - Incubation: | 1 heure à 37°C | |
| - 3 lavages | | |
| Conjugué monoclonal (IgG toxo) utilisé au | | 1/32000 pour HspA |
| | | 1/64000 pour N6flbA- |
| | | 1/56000 pour N6 |
| | volume déposé | 100 µl |
| - Incubation du conjugué 1 heure à 37°C | | |
| - 4 lavages | | |
| - Révélation de la réaction enzymatique par OPD + substrat 30 minutes à l'obscurité | | |
| - Arrêt de la réaction enzymatique par ⁻H₂SO₄ | | |
| - Lecture des D.O. à 492 nm / 620 nm. | | |
| Conversion desD.O en Unités Arbitraires (UA). | | |

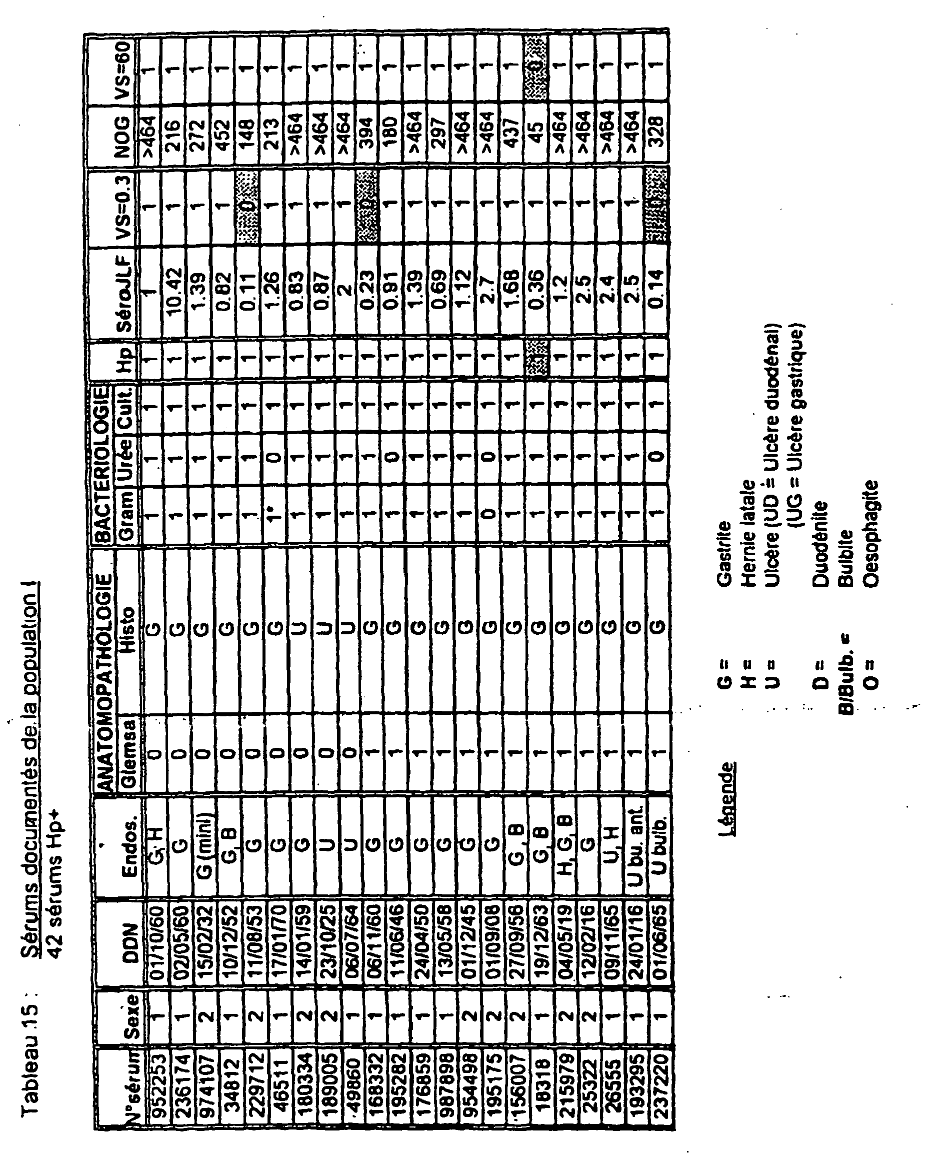

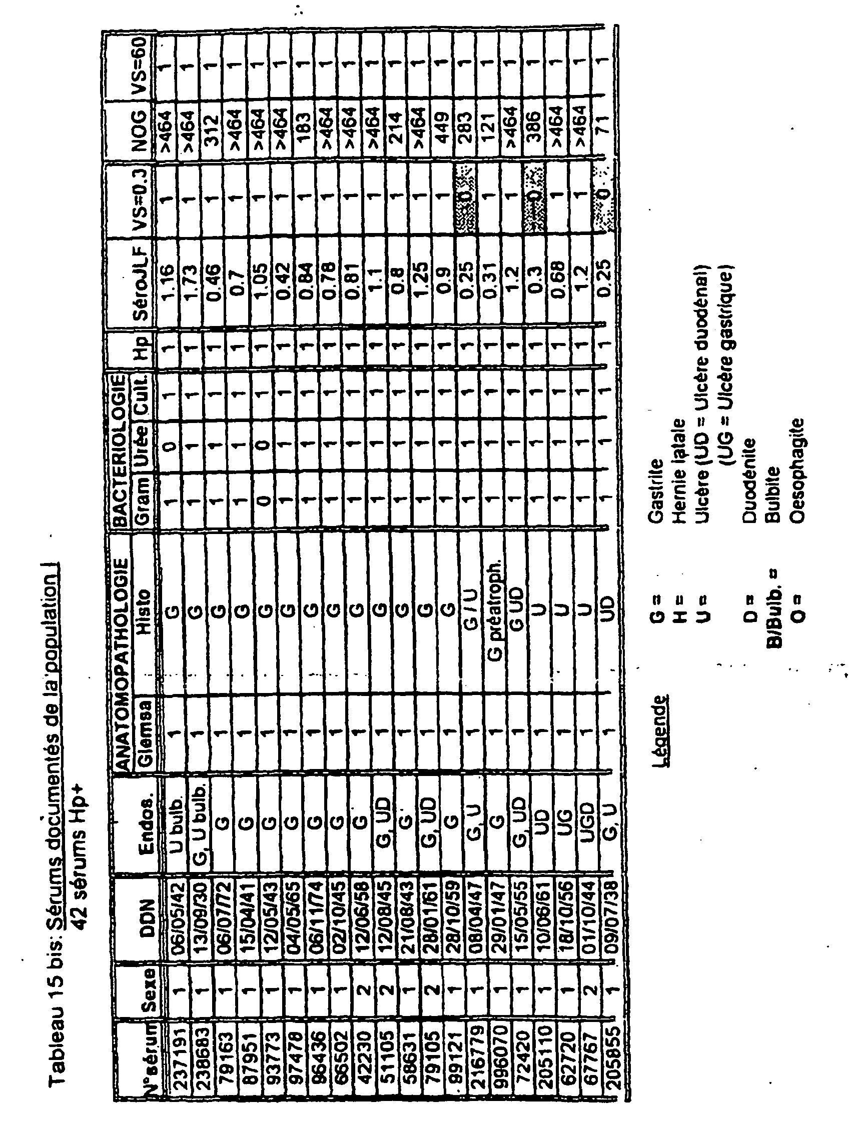

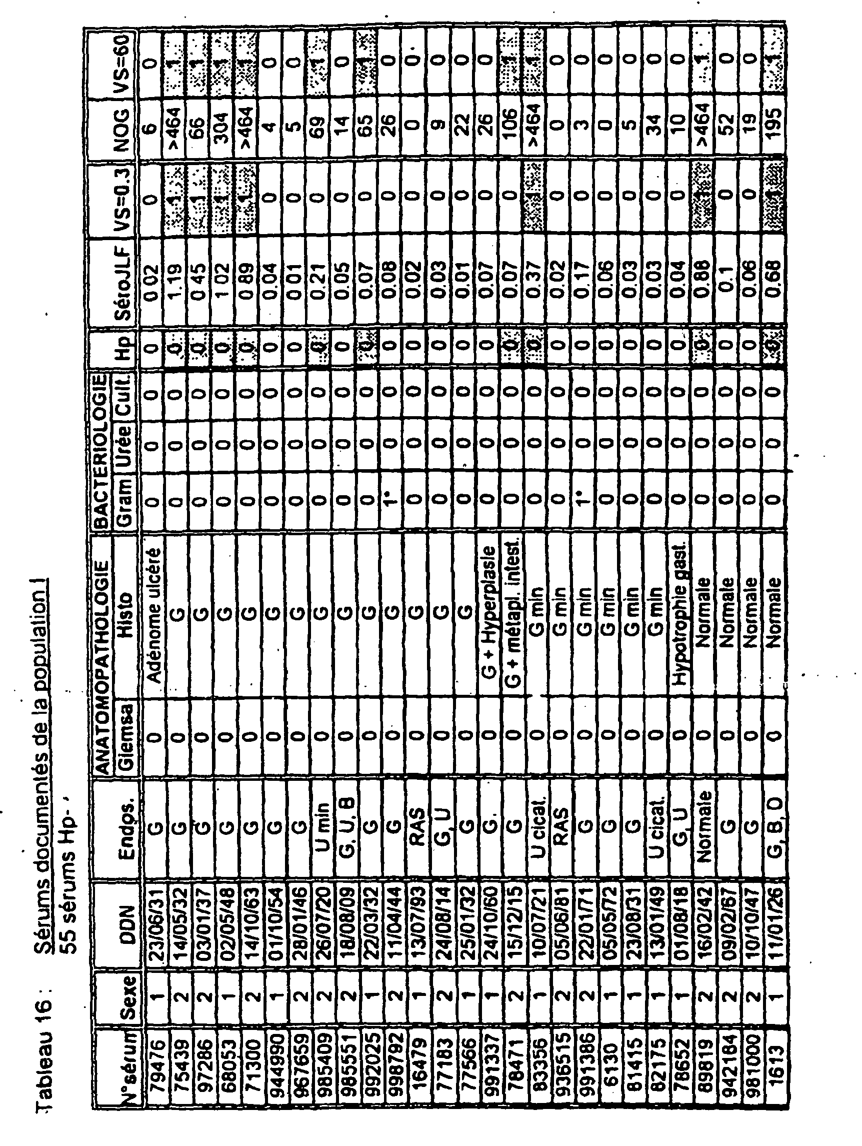

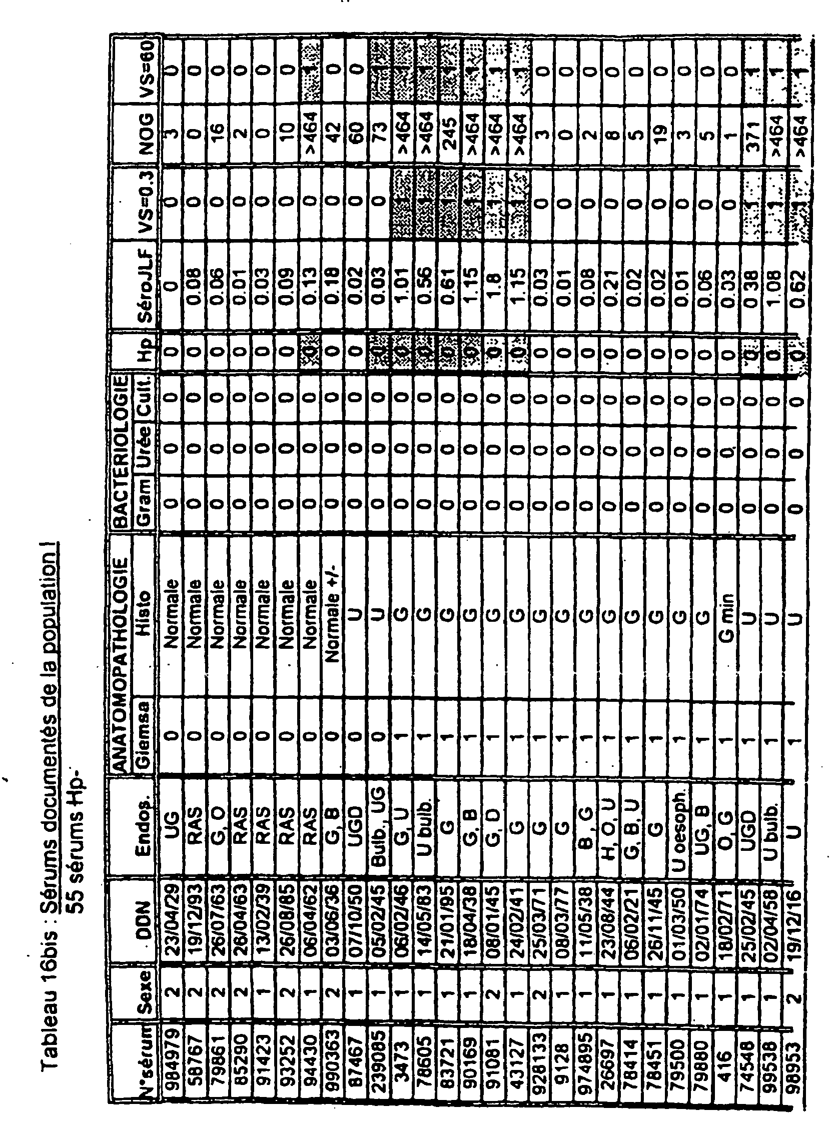

**Tableau 17 : Population documentée de la population I 55 sérums Hp- 42 sérums Hp+**

| | **SENSIBILITE** | **SPECIFICITE** |
|---|---|---|
| Séro JLF | 85.7% (36/42) | 70.9% (39/55) |
| NOG 60 | 97.8% (41/42) | 61.8% (34/55) |

### PROTOCOLES D'EXTRACTION A PARTIR DE LA SOUCHE AFFLAGELLEE N6flbA-.

Quantité fournie 800 mg de bactéries recueillies avec du PBS, centrifugées

### 3 extractions testées.

### EXTRACTIONS DE LA SOUCHE AFLAGELLEE

| | Extraction Glycine | Extraction N-Octyl Glucoside | Extraction PBS |
|---|---|---|---|
| Récupération | PBS | PBS 0.01 M | PBS pH 7.4 |
| Lavage | 2 fois dans du PBS | 2 fois dans du PBS | |
| | 8.000 rpm / 12 min | 8.000 rpm / 12 min | |
| Extraction | Tampon Glycine Acide | PBS contenant : | Vortexer pendant 1 min. |
| | 0.2M pH 2.2 15 min. Tp ambiante | 1% N-Octyl Glucoside pH 7.2 | |
| | agitation douce | (Sigma Chemical Co.) | |
| | 100 mg (poids humide) pour 2.5 ml | 20 min. Tp ambiante | |
| Centrifugation | 11.000g | 23.500 g | 5.000 g |
| | 15 min. | 20 min. | 10 min. |
| Neutralisation | NaOH 1M | | |
| Dialyse | PBS pH 7.2 | PBS pH 7.2 | PBS pH 7.2 |
| | 24 H à +4°C | 24 H à +4°C | 24 H à +4°C |
| | cut-off 10.000 | cut-off 10.000 | cut-oft 10.000 |
| Conservation | détermination de la concentration Conservation à -20°C | élimination des particules insolubles conservation à -20°C | détermination de la concentration Conservation à - 20°C |

### SDS PAGE DES DIFFERENTS EXTRAITS DE LA SOUCHE AFLAGELLEE N6 FLBA-

| N° puits | Type d'échantillon | Concentration ug/ml | Volume échantillon / Volume à Tampon | Volume déposé |
|---|---|---|---|---|
| 1 | Etalon PM | | 5+5/190 | 10 |
| 2 | Extrait Glycine | 202.9 | 60/60 | 60 |
| 3 | | | | |
| 4 | Extrait N-Octyl Glucoside | 874 | 51/39 | 60 |
| 5 | | | | |
| 6 | Extrait PBS 1 | 539.2 | 60/20 | 60 |
| 7 | | | | |
| 8 | Extrait PBS 2 | 77.9 | 60 / 20 | 60 |
| 9 | | | | |
| 10 | Etalon PM | | 5+5/190 | 10 |
| 11 | Culot Extrait Glycine | 2778.7 | 20/20 | 20 |
| 12 | | | | |
| 13 | Culot Extrait Glucoside | 972.9 | 40/40 | 60 |
| 14 | | | | |
| 15 | Extrait Glycine sédimenté | 309.3 | 60/20 | 60 |
| 16 | | | | |
| 17 | HspA Mal E | 3000 | 20/20 | 20 |
| 18 | | | | |
| 19 | | | | |
| 20 | Kaléidoscope | | | 20 |

### Références :

1. Andrews, G.P., Maurelli, A.T. : mxiA of Shigella flexneri 2a, which facilitates export of invasion plasmid antigens, encodes a homolog of the low-calcium-response protein. LcrD of Yersinia pestis. Infect. Immun. 60: 3287-3295 (1992).
2. Galan, J.E., Ginocchio, C. Costeas, P. : Molecular and functional characterization of the Salmonella invasion gene invA : homology of InvA to members of a new protein family. J. Bacteriol. 174, 4338-4349 (1992.
3. Leying, H., Suerbaum, S. Geis, G., Haas, R. : Cloning and genetic characterization of a Helicobacter pylori flagellin gene. Mol. Microbiol. 6. 2563-2874 (1993).
5. O'Toole, P.W., Kostrzynska, M., Trust, T.J.: Non-mobile mutants of Helicobacter pylori and Helicobacter mustelae defective in flagellar hook production. Mol. Microbiol. 14,691-703 (1994).
6. Plano, G.V., Barve, S.S., Straley, S.C. : LcrD, a membrane-bound regulator of the Yersinia pestis low-calcium response. J. Bacteriol. 173. 7293-7303 (1991).
7. Ramakrishnaan, G., Zhao, J-L., Newton, A. : The cell cycle-regulated gene flbF of Caulobacter crescentus is homologous to a virulence locus of Yersinia pestis. J. Bacteriol. 173, 7283-7292 (1991).
8. Suerbaum, S., Josenhans, C., Labigne, A.: Cloning and genetic characterization of the Helicobacter pylori and Helicobacter mustelae flab flagellin genes and construction of H. pylori flaA- and flaB-negative mutants by electroporation-mediated allelic exchange, J. Bacteriol. 175, 3278-3288 (1993).

## Revendications

1. Utilisation pour la détection *in vitro* d'une infection par H.pylori dans un échantillon de fluide biologique prélevé chez un patient, d'une souche bactérienne recombinante d'Helicobacter pylori, ayant un phénotype aflagellé résultant de la mutation par substitution, addition et/ou délétion de bases ou d'un fragment nucléotidique dans la séquence nucléotidique du gène flbA participant à la régulation de la biosynthèse des protéines des flagelles de H. pylori, ladite séquence nucléotidique du gène flbA hybridant dans des conditions stringentes avec une sonde correspondant à un fragment de nucléotides de H. pylori amplifié à l'aide des deux oligonucléotides dégénérés suivants :
OLFlba-1 : ATGCCTCGAGGTCGAAAAGCAAGATG et OLFlba-2 : GAAATCTTCATACTGGCAGCTCCAGTC, et ladite mutation étant telle que, soit le gène flbA n'est plus exprimé dans ladite souche bactérienne recombinante, soit le gène flbA ne permet pas la synthèse des flagelles de H. pylori.

2. Utilisation selon la revendication 1, dans laquelle la souche bactérienne recombinante est **caractérisée en ce que** le phénotype aflagellé résulte de la mutation dans la séquence nucléotidique du gène flbAdont la séquence non mutée répond à l'enchaînement nucléotidique suivant ou code pour la protéine répondant à la séquence d'acides aminés suivante :

1                                                    31
AGC TTT TTT GTG CCA TAC TTT TAA ACT TTA TAT TAT AAT AAG AGA CAA ACA CAC GTA CCA

61                                                   91
AAA TTA AGG CAT TGA TTT TAG ATT ATG GCA AAC GAA CGC TCC AAA TTA GCT TTT AAA AAG
                                M   A   N   E   R   S   K   L   A   F   K   K
121                                                  151
ACT TTC CCT GTC TTT AAA CGC TTC TTG CAA TCC AAA GAC TTA GCC CTT GTG GTC TTT GTG
T   F   P   V   F   K   R   F   L   Q   S   K   D   L   A   L   V   V   F   V
181                                                  211
ATA GCG ATT TTA GCG ATC ATT ATC GTG CCG TTA CCG CCT TTT GTG TTG GAT TTT TTA CTC
I   A   I   L   A   I   I   I   V   P   L   P   P   F   V   L   D   F   L   L
241                                                  271
ACG ATT TCT ATC GCG CTA TCG GTG TTG ATT ATT TTA ATC GGG CTT TAT ATT GAC AAA CCG
T   I   S   I   A   L   S   V   L   I   I   L   I   G   L   Y   I   D   K   P
301                                                  331
ACT GAT TTT AGC GCT TTC CCC ACT TTA TTA CTC ATT GTA ACC TTA TAC CGC TTG GCT TTA
T   D   F   S   A   F   P   T   L   L   L   I   V   T   L   Y   R   L   A   L
361                                                  391
AAT GTC GCC ACC ACT AGA ATG ATT TTA ACC CAA GGC TAT AAA GGG CCT AGC GCG GTG AGC
N   V   A   T   T   R   M   I   L   T   Q   G   Y   K   G   P   S   A   V   S
421                                                  451
ATT ATT ATC ACG GCG TTT GGG GAA TTT AGC GTG AGC GGG AAT TAT GTG ATT GGG GCT ATT
I   I   I   T   A   F   G   E   F   S   V   S   G   N   Y   V   I   G   A   I
481                                                  511
ATC TTT AGT ATT TTA GTG GTG GTG AAT TTA TTA GTG GTT ACT AAT GGT TCT ACT AGG GTT
I   F   S   I   L   V   V   V   N   L   L   V   V   T   N   G   S   T   R   V
541                                                  571
ACT GAA GTT AGG GCG CGA TTT GCC CTA GAC GCT ATG CCA GGA AAG CAA ATG GCG ATT GAT
T   E   V   R   A   R   F   A   L   D   A   M   P   G   K   Q   M   A   I   D
601                                                  631
GCG GAT TTA AAT TCA GGG CTT ATT GAT GAT AAG GAA GCT AAA AAA CGG CGC GCC GCT CTA
A   D   L   N   S   G   L   I   D   D   K   E   A   K   K   R   R   A   A   L
661                                                  691
AGC CAA GAA GCG GAT TTT TAT GGT GCG ATG GAT GGG GCG TCT AAA TTT GTC AAA GGC GAT
S   Q   E   A   D   F   Y   G   A   M   D   G   A   S   K   F   V   K   G   D
721                                                  751
GCG ATC GCT TCT ATC ATT ATC ACG CTT ATC AAT ATC ATT GGG GGT TTT TTA GTG GGC GTG
A   I   A   S   I   I   I   T   L   I   N   I   I   G   G   F   L   V   G   V
781                                                  811
TTC CAA AGG GAT ATG AGC TTG AGC TTT AGT GCT AGC ACT TTC ACT ATC TTA ACC ATT GGC
F   Q   R   D   M   S   L   S   F   S   A   S   T   F   T   I   L   T   I   G
841                                                  871
GAT GGG CTT GTA GGG CAA ATC CCT GCC TTA ATC ATT GCG ACA CGG ACC GGT ATT GTC GCC
D   G   L   V   G   Q   I   P   A   L   I   I   A   T   R   T   G   I   V   A
901                                                  931
ACT CGC ACC ACG CAA AAC GAA GAA GAG GAC TTT GCT TCT AAG CTC ATC ACA CAG CTC ACC
T   R   T   T   Q   N   E   E   E   D   F   A   S   K   L   I   T   Q   L   T
961                                                  991
AAT AAA AGC AAA ACT TTA GTG ATT GTG GGG GCG ATT TAT TGC TTT TGC ACC ATT CCT GGA
N   K   S   K   T   L   V   I   V   G   A   I   Y   C   F   C   T   I   P   G
1021                                                 1051
CTC CCT ACC TTT TCT TTA GCG TTT GTA GGG GCT CTC TTT TTA TTC ATC GCA TGG CTG ATT
L   P   T   F   S   L   A   F   V   G   A   L   F   L   F   I   A   W   L   I
1081                                                 1111
AGC AGG GAG GGA AAG GAC GGG TTG CTC ACT AAA TTA GAA AAT TAT TTG AGT CAA AAA TTC
S   R   E   G   K   D   G   L   L   T   K   L   E   N   Y   L   S   Q   K   F
1141                                                 1171
GGC TTG GAT TTG AGC GAA AAA CCC CAC AGC TCC AAA ATC AAA CCC CAC GCC CCC ACC ACA
G   L   D   L   S   E   K   P   H   S   S   K   I   K   P   H   A   P   T   T
1201                                                 1231
AGG GCT AAA ACC CAA GAA GAG ATT AAA AGA GAA GAA GAG CAA GCC ATT GAT GAA GTG TTA
R   A   K   T   Q   E   E   I   K   R   E   E   E   Q   A   I   D   E   V   L

1261                                                    1291
AAA ATT GAA TTT TTA GAA TTG GCT TTA GGC TAT CAG CTC TAC AGC TTA GCG GAC ATG AAA
K   I   E   F   L   E   L   A   L   G   Y   Q   L   Y   S   L   A   D   M   K
1321                                                    1351
CAA GGG GGC GAT TTG TTA GAA AGG ATT AGG GGT ATT AGA AAA AAG ATA GCG AGC GAT TAT
Q   G   G   D   L   L   E   R   I   R   G   I   R   K   K   I   A   S   D   Y
1381                                                    1411
GGT TTT TTG ATG CCT CAA ATT AGG ATT AGG GAT AAT TTA CAA CTC CCC CCA ACG CAT TAT
G   F   L   M   P   Q   I   R   I   R   D   N   L   Q   L   P   P   T   H   Y
1441                                                    1471
GAA ATC AAG CTT AAG GGC ATT GTG ATT GGT GAA GGC ATG GTG ATG CCG GAT AAG TTT TTA
E   I   K   L   K   G   I   V   I   G   E   G   M   V   M   P   D   K   F   L
1501                                                    1531
GCC ATG AAT ACC GGT TTT GTG AAT AAA GAA ATT GAA GGC ATT CCT ACT AAA GAG CCG GCT
A   M   N   T   G   F   V   N   K   E   I   E   G   I   P   T   K   E   P   A
1561                                                    1591
TTT GGA ATG GAC GCT TTA TGG ATT GAA ACT AAA AAT AAA GAA GAA GCC ATC ATT CAA GGC
F   G   M   D   A   L   W   I   E   T   K   N   K   E   E   A   I   I   Q   G
1621                                                    1651
TAT ACC ATT ATT GAT CCA AGC ACC GTT ATT GCG ACG CAC ACC AGC GAA TTA GTG AAA AAA
Y   T   I   I   D   P   S   T   V   I   A   T   H   T   S   E   L   V   K   K
1681                                                    1711
TAC GCT GAA GAT TTT ATC ACT AAA GAT GAA GTG AAA TCC CTT TTA GAG CGC TTG GCC AAA
Y   A   E   D   F   I   T   K   D   E   V   K   S   L   L   E   R   L   A   K
1741/581                                                1771
GAC TAT CCT ACG ATT GTA GAA GAG AGT AAA AAA ATC CCC ACC GGT GCG ATC CGA TCA GTG
D   Y   P   T   I   V   E   E   S   K   K   I   P   T   G   A   I   R   S   V
1801                                                    1831
TTG CAA GCC TTG TTG CAT GAA AAA ATC CCC ATT AAA GAC ATG CTC ACT ATT TTA GAA ACG
L   Q   A   L   L   H   E   K   I   P   I   K   D   M   L   T   I   L   E   T
1861                                                    1891
ATT ACC GAT ATT GCG CCA TTA GTT CAA AAC GAT GTG AAT ATC TTA ACC GAA CAA GTG AGG
I   T   D   I   A   P   L   V   Q   N   D   V   N   I   L   T   E   Q   V   R
1921                                                    1951
GCG AGG CTT TCT AGG GTG ATC ACT AAC GCT TTT AAA TCT GAA GAC GGG CGT TTG AAA TTT
A   R   L   S   R   V   I   T   N   A   F   K   S   E   D   G   R   L   K   F
1981                                                    2011
TTA ACC TTT TCT ACC GAT AGC GAA CAA TTT TTG CTT AAT AAA TTG CGA GAA AAT GGC ACT
L   T   F   S   T   D   S   E   Q   F   L   L   N   K   L   R   E   N   G   T
2041                                                    2071
TCT AAG AGC CTA CTA CTC AAT GTG GGC GAA TTG CAA AAA CTC ATT GAA GCG GTC TCT GAA
S   K   S   L   L   L   N   V   G   E   L   Q   K   L   I   E   A   V   S   E
2101                                                    2131
GAG GCC ATG AAA GTC TTG CAA AAA GGG ATC GCT CCG GTG ATT TTG ATC GTA GAG CCT AAT
E   A   M   K   V   L   Q   K   G   I   A   P   V   I   L   I   V   E   P   N
2161                                                    2191
TTA AGA AAA GCC CTT TCT AAT CAA ATG GAG CAG GCT AGG ATT GAT GTA ATC GTG CTA AGC
L   R   K   A   L   S   N   Q   M   E   Q   A   R   I   D   V   I   V   L   S
2221                                                    2251
CAT GCT GAA TTA GAT CCT AAC TCT AAT TTT GAA GCC TTA GGC ACG ATC CAT ATT AAC TTT
H   A   E   L   D   P   N   S   N   F   E   A   L   G   T   I   H   I   N   F
2281                                                    2311
TAA GGG ATA AAT AAT TGA TAA AAA AGG AGA ATG ATG CAA GTT TAT CAC CTT TCA CAC ATT
*
2341                                                    2371
GAT TTA GAC GGC TAT GCA TGC CAG CTT GTT TCA AAA CAA TTT TTT AAA AAT ATC CAA TGC
2401                                                    2431
TAT AAC GCT AAT TAC GGG CGT GAA GTC TCA GCG AGA ATT TAT GAG ATT TTA AAC GCG ATC
2461                                                    2491
GCT CAA TCT AAA GAG AGT GAA TTC CTT ATT TTG ATT AGC GA

3. Utilisation selon la revendication 1 ou la revendication 2, dans laquelle la souche bactérienne recombinante est **caractérisée en ce qu'**elle est en outre dépourvue de la protéine du crochet de H. pylori.

4. Souches bactériennes recombinantes, **caractérisées en ce qu'**elles sont obtenues par mutation, par substitution, addition et/ou délétion de bases ou d'un fragment nucléotidique dans la séquence nucléotidique du gène flbA participant à la régulation de la biosynthèse des protéines des flagelles de H. pylori, ladite séquence hybridant dans des conditions stringentes avec une sonde correspondant à un fragment de nucléotides de H. pylori amplifié à l'aide des deux oligonucléotides dégénérés suivants OLFlba-1
ATGCCTCGAGGTCGAAAAGCAAGATG et OLFlba-2: GAAATCTTCATACTGGCAGCTCCAGTC, et ladite mutation étant telle que, soit le gène flbA n'est plus exprimé dans ladite souche bactérienne recombinante, soit le gène flba ne permet pas la synthèse des flagelles de H. pylori,
ladite mutation étant effectuée dans la souche N6 déposée à la NCIMB le 26 juin 1992 sous le numéro NCIMB 40512.

5. Souche bactérienne recombinante selon la revendication 4, **caractérisée en ce qu'**il s'agit de la souche N6flbA - , déposée à la NCIMB le 30 juin 1995 sous le numéro NCIMB 40747.

6. Utilisation selon l'une des revendications 1 à 3, dans laquelle la souche bactérienne est **caractérisée en ce qu'**elle est en outre mutée de façon à produire une uréase atténuée, voire à ne plus produire d'uréase, la mutation consistant par exemple en une mutation de la séquence nucléotidique d'un ou plusieurs gènes choisis parmi les gènes ureA, ureB, ureC, ureD, ureE, ureF, ureG, ureH ou ureI.

7. Souche bacterienne recombinante selon la revendication 4 ou 5 **caractérisée en ce qu'**elle est en outre mutée de façon à produire une uréase atténuée, voire à ne plus produire d'uréase, la mutation consistant par exemple en une mutation de la séquence nucléotidique d'un ou plusieurs gènes choisis parmi les gènes ureA, ureB, ureC, ureD, UreE, UreF, ureG, ureH, ou ureI.

8. Utilisation pour la détection *in vitro* d'une infection par H.pylori dans un échantillon de fluide biologique prélevé chez un patient, d'un extrait bactérien **caractérisé en ce qu'**il s'agit d'un extrait de souche bactérienne définie dans l'une quelconque des revendications 1 à 3 ou 6.

9. Utilisation selon la revendication 8 d'un extrait bactérien , ledit extrait étant **caractérisé en ce qu'**il est obtenu après une extraction au N-OctylGlucoside.

10. Utilisation selon la revendication 8 d'un extrait bactérien, ledit extrait étant **caractérisé en ce qu'**il est obtenu après une extraction au PBS ou à la glycine.

11. Extrait bactérien **caractérisé en ce qu'**il s'agit d'un extrait de souches bactériennes selon l'une quelconque des revendications 4 ou 5.

12. Extrait bactérien selon la revendication 11 **caractérisé en ce qu'**il est obtenu après une extraction au N-OctylGlucoside.

13. Extrait bactérien selon la revendication 11 **caractérisé en ce qu'**il est obtenu après une extraction au PBS ou à la glycine.

14. Procédé de détection in vitro d'une infection par H. pylori dans un échantillon de fluide biologique prélevé chez un patient, notamment dans un échantillon de sérum, comprenant les étapes de :
- mise en contact de l'échantillon testé avec une souche bactérienne définie dans l'une quelconque des revendications 1 à 6, ou avec un extrait bactérien défini dans l'une quelconque des revendications 8 à 10,
- détection d'une réaction immunologique entre ladite souche bactérienne et des anticorps dirigés contre H. pylori, présents dans l'échantillon testé.

15. Utilisation, pour l'obtention d'anticorps contre H. pylori, d'une souche bactérienne définie dans l'une quelconque des revendications 1 à 6, ou d'un extrait bactérien défini dans l'une quelconque des revendications 8 à 10.

16. Utilisation selon la revendication 15 pour la préparation d'une composition vaccinante pour l'obtention d'anticorps protecteurs contre une infection par H. pylori.

17. Sérum polyclonal dirigé contre une souche de H. pylori selon l'une quelconque des revendications 4 ou 5.

18. Utilisation pour la détection in vitro d'une infection par H. pylori dans un échantillon biologique, d'une composition comprenant à titre de principe actif un sérum polyclonal obtenu contre une souche de H. pylori de phénotype aflagellé définie dans l'une quelconque des revendications 1 à 6.

19. Coffret de diagnostic d'anticorps de patients infectés par H. pylori comprenant une souche bactérienne définie dans l'une des revendications 1 à 6 ou un extrait bactérien défini dans l'une quelconque des revendications 8 à 10, et des réactifs nécessaires pour mettre en évidence une réaction de type antigène/anticorps

## Claims

1. Use, for the *in vitro* detection of an infection with H. pylori in a sample of biological fluid taken from a patient, of a recombinant bacterial strain of Helicobacter pylori, having an aflagellate phenotype resulting from mutation by substitution, addition and/or deletion of bases or of a nucleotide fragment in the nucleotide sequence of the flbA gene which participates in the regulation of biosynthesis of the H. pylori flagellar proteins, said nucleotide sequence of the flbA gene hybridizing, under stringent conditions, with a probe corresponding to an H. pylori nucleotide fragment amplified using the following two degenerate oligonucleotides:
OLFlba-1: ATGCCTCGAGGTCGAAAAGCAAGATG and OLFlba-2: GAAATCTTCATACTGGCAGCTCCAGTC, and said mutation being such that either the flbA gene is no longer expressed in said recombinant bacterial strain, or the flbA gene does not allow the synthesis of the H. pylori flagellae.

2. Use according to Claim 1, in which the recombinant bacterial strain is **characterized in that** the aflagellate phenotype results from mutation in the nucleotide sequence of the flbA gene, the non-mutated sequence of which corresponds to the following nucleotide chain or encodes the protein corresponding to the following amino acid sequence:

1
AGC TTT TTT GTG CCA TAC TTT TAA ACT TTA TAT TAT AAT AAG AGA CAA ACA CAC CTA CCA
61
AAA TTA AGG CAT TGA TTT TAG ATT ATG GCA AAC GAA CGC TCC AAA TTA GCT TTT AAA AAG
M A N E R S K L A F K K
121
ACT TTC CCT GTC TTT AAA CGC TTC TTG CAA TCC AAA GAC TTA GCC CTT GTG GTC TTT GTG
T F P V F K R F L Q S K D L A L V V F V
181
ATA GCG ATT TTA GCG ATC ATT ATC GTG CCG TTA CCG CCT TTT GTG TTG GAT TTT TTA CTC
I A I L A I I I V P L P P F V L D F L L
241
ACG ATT TCT ATC GCG CTA TCG GTG TTG ATT ATT TTA ATC GGG CTT TAT ATT GAC AAA CCG
T I S I A L S V L I I L I G L Y I D K P
301
ACT GAT TTT AGC GCT TTC CCC ACT TTA TTA CTC ATT GTA ACC TTA TAC CGC TTG GCT TTA
T D F S A F P T L L L I V T L Y R L A L
361
AAT GTC GCC ACC ACT AGA ATG ATT TTA ACC CAA GGC TAT AAA GGG CCT AGC GCG GTG AGC
N V A T T R M I L T Q G Y K G P S A V S
421
ATT ATT ATC ACG GCG TTT GGG GAA TTT AGC GTG AGC GGG AAT TAT GTG ATT GGG GCT ATT
I I I T A F G E F S V S G N Y V I G A I
481
ATC TTT AGT ATT TTA GTG GTG GTG AAT TTA TTA GTG GTT ACT AAT GGT TCT ACT AGG GTT
I F S I L V V V N L L V V T N G S T R V
541
ACT GAA GTT AGG GCG CGA TTT GCC CTA GAC GCT ATG CCA GGA AAG CAA ATG GCG ATT GAT
T E V R A R F A L D A M P G K Q M A I D
601
GCG GAT TTA AAT TCA GGG CTT ATT GAT GAT AAG GAA GCT AAA AAA CGG CGC GCC GCT CTA
A D L N S G L I D D K E A K K R R A A L
661
AGC CAA GAA GCG GAT TTT TAT GGT GCG ATG GAT GGG GCG TCT AAA TTT GTC AAA GGC GAT
S Q E A D F Y G A M D G A S K F V K G D
721
GCG ATC GCT TCT ATC ATT ATC ACG CTT ATC AAT ATC ATT GGG GGT TTT TTA GTG GGC GTG
A I A S I I I T L I N I I G G F L V G V
781
TTC CAA AGG GAT ATG AGC TTG AGC TTT AGT GCT AGC ACT TTC ACT ATC TTA ACC ATT GGC
F Q R D M S L S F S A S T F T I L T I G
841
GAT GGG CTT GTA GGG CAA ATC CCT GCC TTA ATC ATT GCG ACA CGG ACC GGT ATT GTC GCC
D G L V G Q I P A L I I A T R T G I V A
901
ACT CGC ACC ACG CAA AAC GAA GAA GAG GAC TTT GCT TCT AAG CTC ATC ACA CAG CTC ACC
T R T T Q N E E E D F A S K L I T Q L T
961
AAT AAA AGC AAA ACT TTA GTG ATT GTG GGG GCG ATT TAT TGC TTT TGC ACC ATT CCT GGA
N K S K T L V I V G A I Y C F C T I P G
1021
CTC CCT ACC TTT TCT TTA GCG TTT GTA GGG GCT CTC TTT TTA TTC ATC GCA TGG CTG ATT
L P T F S L A F V G A L F L F I A W L I
1081
AGC AGG GAG GGA AAG GAC GGG TTG CTC ACT AAA TTA GAA AAT TAT TTG AGT CAA AAA TTC
S R E G K D G L L T K L E N Y L S Q K F
1141
GGC TTG GAT TTG AGC GAA AAA CCC CAC AGC TCC AAA ATC AAA CCC CAC GCC CCC ACC ACA
G L D L S E K P H S S K I K P H A P T T
1201
AGG GCT AAA ACC CAA GAA GAG ATT AAA AGA GAA GAA GAG CAA GCC ATT GAT GAA GTG TTA
R A K T Q E E I K R E E E Q A I D E V L

1261                                                    1291
AAA ATT GAA TTT TTA GAA TTG GCT TTA GGC TAT CAG CTC TAC AGC TTA GCG GAC ATG AAA
K   I   E   F   L   E   L   A   L   G   Y   Q   L   Y   S   L   A   D   M   K
1321                                                    1351
CAA GGG GGC GAT TTG TTA GAA AGG ATT AGG GGT ATT AGA AAA AAG ATA GCG AGC GAT TAT
Q   G   G   D   L   L   E   R   I   R   G   I   R   K   K   I   A   S   D   Y
1381                                                    1411
GGT TTT TTG ATG CCT CAA ATT AGG ATT AGG GAT AAT TTA CAA CTC CCC CCA ACG CAT TAT
G   F   L   M   P   Q   I   R   I   R   D   N   L   Q   L   P   P   T   H   Y
1441                                                    1471
GAA ATC AAG CTT AAG GGC ATT GTG ATT GGT GAA GGC ATG GTG ATG CCG GAT AAG TTT TTA
E   I   K   L   K   G   I   V   I   G   E   G   M   V   M   P   D   K   F   L
1501                                                    1531
GCC ATG AAT ACC GGT TTT GTG AAT AAA GAA ATT GAA GGC ATT CCT ACT AAA GAG CCG GCT
[?] [?] N   [?] G   [?] [?] N   K   E   I   E   G   I   [?] [?] [?] [?] [?] [?]
1561                                                    1591
TTT GGA ATG GAC GCT TTA TGG ATT GAA ACT AAA AAT AAA GAA GAA GCC ATC ATT CAA GGC
F   G   M   D   A   L   W   I   E   T   K   N   K   E   E   A   I   I   Q   G
1621                                                    1651
TAT ACC ATT ATT GAT CCA AGC ACC GTT ATT GCG ACG CAC ACC AGC GAA TTA GTG AAA AAA
Y   T   I   I   D   P   S   T   V   I   A   T   H   T   S   E   L   V   K   K
1681                                                    1711
TAC GCT GAA GAT TTT ATC ACT AAA GAT GAA GTG AAA TCC CTT TTA GAG CGC TTG GCC AAA
Y   A   E   D   F   I   T   K   D   E   V   K   S   L   L   E   R   L   A   K
1741/581                                                1771
GAC TAT CCT ACG ATT GTA GAA GAG AGT AAA AAA ATC CCC ACC GGT GCG ATC CGA TCA GTG
D   Y   P   T   I   V   E   E   S   K   K   I   P   T   G   A   I   R   S   V
1801                                                    1831
TTG CAA GCC TTG TTG CAT GAA AAA ATC CCC ATT AAA GAC ATG CTC ACT ATT TTA GAA ACG
L   Q   A   L   L   H   E   K   I   P   I   K   D   M   L   T   I   L   E   T
1861                                                    1891
ATT ACC GAT ATT GCG CCA TTA GTT CAA AAC GAT GTG AAT ATC TTA ACC GAA CAA GTG AGG
I   T   D   I   A   P   L   V   Q   N   D   V   N   I   L   T   E   Q   V   R
1921                                                    1951
GCG AGG CTT TCT AGG GTG ATC ACT AAC GCT TTT AAA TCT GAA GAC GGG CGT TTG AAA TTT
A   R   L   S   R   V   I   T   N   A   F   K   S   E   D   G   R   L   K   F
1981                                                    2011
TTA ACC TTT TCT ACC GAT AGC GAA CAA TTT TTG CTT AAT AAA TTG CGA GAA AAT GGC ACT
L   T   F   S   T   D   S   E   Q   F   L   L   N   K   L   R   E   N   G   T
2041                                                    2071
TCT AAG AGC CTA CTA CTC AAT GTG GGC GAA TTG CAA AAA CTC ATT GAA GCG GTC TCT GAA
S   K   S   L   L   L   N   V   G   E   L   Q   K   L   I   E   A   V   S   E
2101                                                    2131
GAG GCC ATG AAA GTC TTG CAA AAA GGG ATC GCT CCG GTG ATT TTG ATC GTA GAG CCT AAT
E   A   M   K   V   L   Q   K   G   I   A   P   V   I   L   I   V   E   P   N
2161                                                    2191
TTA AGA AAA GCC CTT TCT AAT CAA ATG GAG CAG GCT AGG ATT GAT GTA ATC GTG CTA AGC
L   R   K   A   L   S   N   Q   M   E   Q   A   R   I   D   V   I   V   L   S
2221                                                    2251
CAT GCT GAA TTA GAT CCT AAC TCT AAT TTT GAA GCC TTA GGC ACG ATC CAT ATT AAC TTT
H   A   E   L   D   P   N   S   N   F   E   A   L   G   T   I   H   I   N   F
2281                                                    2311
TAA GGG ATA AAT AAT TGA TAA AAA AGG AGA ATG ATG CAA GTT TAT CAC CTT TCA CAC ATT
*
2341                                                    2371
GAT TTA GAC GGC TAT GCA TGC CAG CTT GTT TCA AAA CAA TTT TTT AAA AAT ATC CAA TGC
2401                                                    2431
TAT AAC GCT AAT TAC GGG CGT GAA GTC TCA GCG AGA ATT TAT GAG ATT TTA AAC GCG ATC
2461                                                    2491
GCT CAA TCT AAA GAG AGT GAA TTC CTT ATT TTG ATT AGC GA

3. Use according to Claim 1 or Claim 2, in which the recombinant bacterial strain is **characterized in that** it also lacks the H. pylori hook protein.

4. Recombinant bacterial strains, **characterized in that** they are obtained by mutation, by substitution, addition and/or deletion of bases or of a nucleotide fragment in the nucleotide sequence of the flbA gene which participates in the regulation of biosynthesis of the H. pylori flagellar proteins, said sequence hybridizing, under stringent conditions, with a probe corresponding to an H. pylori nucleotide fragment amplified using the following two degenerate oligonucleotides:
OLFlba-1: ATGCCTCGAGGTCGAAAAGCAAGATG and OLFlba-2: GAAATCTTCATACTGGCAGCTCCAGTC, and said mutation being such that either the flbA gene is no longer expressed in said recombinant bacterial strain, or the flbA gene does not allow the synthesis of the H. pylori flagellae, said mutation being effected in the N6 strain deposited with the NCIMB on 26 June 1992 under the number NCIMB 40512.

5. Recombinant bacterial strain according to Claim 4, **characterized in that** it is the N6flbA - strain, deposited with the NCIMB on 30 June 1995 under the number NCIMB 40747.

6. Use according to one of Claims 1 to 3, in which the bacterial strain is **characterized in that** it is also mutated so as to produce an attenuated urease, or even so as to no longer produce urease, the mutation consisting, for example, of a mutation of the nucleotide sequence of one or more genes chosen from the ureA, ureB, ureC, ureD, ureE, ureF, ureG, ureH or ureI genes.

7. Recombinant bacterial strain according to Claim 4 or 5, **characterized in that** it is also mutated so as to produce an attenuated urease, or even so as to no longer produce urease, the mutation consisting, for example, of a mutation of the nucleotide sequence of one or more genes chosen from the ureA, ureB, ureC, ureD, ureE, ureF, ureG, ureH or ureI genes.

8. Use, for the *in vitro* detection of an infection with H. pylori in a sample of biological fluid taken from a patient, of a bacterial extract **characterized in that** it is an extract of a bacterial strain defined in any one of Claims 1 to 3 or 6.

9. Use, according to Claim 8, of a bacterial extract, said extract being **characterized in that** it is obtained after an extraction with N-octylglucoside.

10. Use, according to Claim 8, of a bacterial extract, said extract being **characterized in that** it is obtained after an extraction with PBS or with glycine.

11. Bacterial extract, **characterized in that** it is an extract of bacterial strains according to either one of Claims 4 and 5.

12. Bacterial extract according to Claim 11, **characterized in that** it is obtained after an extraction with N-octylglucoside.

13. Bacterial extract according to Claim 11, **characterized in that** it is obtained after an extraction with PBS or with glycine.

14. Method for the in vitro detection of an infection with H. pylori in a sample of biological fluid taken from a patient, in particular in a serum sample, comprising the steps consisting in:
- bringing the sample tested into contact with a bacterial strain defined in any one of Claims 1 to 6, or with a bacterial extract defined in any one of Claims 8 to 10,
- detecting an immunoreaction between said bacterial strain and antibodies directed against H. pylori, present in the sample tested.

15. Use, for obtaining antibodies against H. pylori, of a bacterial strain defined in any one of Claims 1 to 6, or of a bacterial extract defined in any one of Claims 8 to 10.

16. Use according to Claim 15, for the preparation of an immunizing composition for obtaining antibodies which protect against an infection with H. pylori.

17. Polyclonal serum directed against a strain of H. pylori according to either one of Claims 4 and 5.

18. Use, for the in vitro detection of an infection with H. pylori in a biological sample, of a composition comprising, as active ingredient, a polyclonal serum obtained against a strain of H. pylori having an aflagellate phenotype defined in any one of Claims 1 to 6.

19. Kit for the diagnosis of antibodies of patients infected with H. pylori, comprising a bacterial strain defined in one of Claims 1 to 6 or a bacterial extract defined in any one of Claims 8 to 10, and reagents required for demonstrating an antigen/antibody type reaction.

## Patentansprüche

1. Verwendung eines rekombinanten Bakterienstamms von Heliobacter pylori zum *in vitro*-Nachweis einer Infektion mit H. pylori in einer Probe einer biologischen Flüssigkeit, die einem Patienten entnommen wurde, wobei der Bakterienstamm einen flagellenlosen Phänotyp aufweist, der aus der Mutation durch Substitution, Addition und/oder Deletion von Basen oder eines Nucleotidfragments in der Nucleotidsequenz des flbA-Gens resultiert, das an der Regulierung der Biosynthese der Flagellenproteine von H. pylori beteiligt ist, und wobei die Nucleotidsequenz des flbA-Gens unter stringenten Bedingungen mit einer Sonde hybridisiert, die einem Nucleotidfragment von H. pylori entspricht, das mithilfe der beiden folgenden degenerierten Oligonucleotide amplifiziert wurde:
OLFlba-1 : ATGCCTCGAGGTCGAAAAGCAAGATG
und
OLFlba- 2 : GAAATCTTCATACTGGCAGCTCCAGTC,
und die Mutation so gestaltet ist, dass entweder das flbA-Gen nicht mehr im rekombinanten Bakterienstamm exprimiert wird, oder das flbA-Gen die Synthese der Flagellen von H-pylori nicht zulässt.

2. Verwendung gemäß Anspruch 1, wobei der rekombinante Bakterienstamm **dadurch gekennzeichnet ist, dass** der flagellenlose Phänotyp aus der Mutation in der Nucleotidsequenz des flbA-Gens resultiert, dessen nicht mutierte Sequenz der folgenden Nucleotidabfolge entspricht oder das Protein mit der folgenden Aminosäurensequenz codiert:

1 31
AGC TTT TTT GTG CCA TAC TTT TAA ACT TTA TAT TAT AAT AAG AGA CAA ACA CAC CTA CCA

61 91
AAA TTA AGG CAT TGA TTT TAG ATT ATG GCA AAC GAA CGC TCC AAA TTA GCT TTT AAA AAG
M A N E R S K L A F K K
121 151
ACT TTC CCT GTG TTT AAA CGC TTC TTG CAA TCC AAA GAC TTA GCC CTT GTG GTC TTT GTG
T F P V F K R F L Q S K D L A L V V F V
181 211
ATA GCG ATT TTA GCG ATC ATT ATC GTG CCG TTA CCG CCT TTT GTG TTG GAT TTT TTA CTC
I A I L A I I I V P L P P F V L D F L L
241 271
ACG ATT TCT ATC GCG CTA TCG GTG TTG ATT ATT TTA ATC GGG CTT TAT ATT GAC AAA CCG
T I S I A L S V L I I L I G L Y I D K P
301 331
ACT GAT TTT AGC GCT TTC CCC ACT TTA TTA CTC ATT GTA ACC TTA TAC CGC TTG GCT TTA
T D F S A F P T L L L I V T L Y R L A L
361 391
AAT GTC GCC ACC ACT AGA ATG ATT TTA ACC CAA GGC TAT AAA GGG CCT AGC GCG GTG AGC
N V A T T R M I L T Q G Y K G P S A V S
421 451
ATT ATT ATC ACG GCG TTT GGG GAA TTT AGC GTG AGC GGG AAT TAT GTG ATT GGG GCT ATT
I I I T A F G E F S V S G N Y V I G A I
481 511
ATC TTT AGT ATT TTA GTG CTG GTG AAT TTA TTA GTG GTT ACT AAT GGT TCT ACT AGG GTT
I F S I L V L V N L L V V T N G S T R V
541 571
ACT GAA GTT AGG GCG CGA TTT GCC CTA GAC GCT ATG CCA GGA AAG CAA ATG GCG ATT GAT
T E V R A R F A L D A M P G K Q M A I D
601 631
GCG GAT TTA AAT TCA GGG CTT ATT GAT GAT AAG GAA GCT AAA AAA CGG CGC GCC GCT CTA
A D L N S G L I D D K E A K K R R A A L
661 691
AGC CAA GAA GCG GAT TTT TAT GGT GCG ATG GAT GGG GCG TCT AAA TTT GTC AAA GGC GAT
S Q E A D F Y G A M D G A S K F V K G D
721 751
GCG ATC GCT TCT ATC ATT ATC ACG CTT ATC AAT ATC ATT GGG GGT TTT TTA GTG GGC GTG
A I A S I I I T L I N I I G G F L V G V
781 811
TTC CAA AGG GAT ATG AGC TTG AGC TTT AGT GCT AGC ACT TTC ACT ATC TTA ACC ATT GGC
F Q R D M S L S F S A S T F T I L T I G
841 871
GAT GGG CTT GTA GGG CAA ATC CCT GCC TTA ATC ATT GCG ACA CGG ACC GGT ATT GTC GCC
D G L V G Q I P A L I I A T R T G I V A
901 931
ACT CGC ACC ACG CAA AAC GAA GAA GAG GAC TTT GCT TCT AAG CTC ATC ACA CAG CTC ACC
T R T T Q N E E E D F A S K L I T Q L T
961 991
AAT AAA AGC AAA ACT TTA GTG ATT GTG GGG GCG ATT TAT TGC TTT TGC ACC ATT CCT GGA
N K S K T L V I V G A I Y C F C T I P G
1021 1051
CTC CCT ACC TTT TCT TTA GCG TTT GTA GGG GCT CTC TTT TTA TTC ATC GCA TGG CTG ATT
L P T F S L A F V G A L F L F I A W L I
1081 1111
AGC AGG GAG GGA AAG GAC GGG TTG CTC ACT AAA TTA GAA AAT TAT TTG AGT CAA AAA TTC
S R E G K D G L L T K L E N Y L S Q K F
1141 1171
GGC TTG GAT TTG AGC GAA AAA CCC CAC AGC TCC AAA ATC AAA CCC CAC GCC CCC ACC ACA
G L D L S E K P H S S K I K P H A P T T
1201 1231
AGG GCT AAA ACC CAA GAA GAG ATT AAA AGA GAA GAA GAG CAA GCC ATT GAT GAA GTG TTA
R A K T Q E E I K R E E E Q A I D E V L

1261                                                    1291
AAA ATT GAA TTT TTA GAA TTG GCT TTA GGC TAT CAG CTC TAC AGC TTA GCG GAC ATG AAA
K   I   E   F   L   E   L   A   L   G   Y   Q   L   Y   S   L   A   D   M   K
1321                                                    1351
CAA GGG GGC GAT TTG TTA GAA AGG ATT AGG GGT ATT AGA AAA AAG ATA GCG AGC GAT TAT
Q   G   G   D   L   L   E   R   I   R   G   I   R   K   K   I   A   S   D   Y
1381                                                    1411
GGT TTT TTG ATG CCT CAA ATT AGG ATT AGG GAT AAT TTA CAA CTC CCC CCA ACG CAT TAT
G   F   L   M   P   Q   I   R   I   R   D   N   L   Q   L   P   P   T   H   Y
1441                                                    1471
GAA ATC AAG CTT AAG GGC ATT GTG ATT GGT GAA GGC ATG GTG ATG CCG GAT AAG TTT TTA
E   I   K   L   K   G   I   V   I   G   E   G   M   V   M   P   D   K   F   L
1501                                                    1531
GCC ATG AAT ACC GGT TTT GTG AAT AAA GAA ATT GAA GGC ATT CCT ACT AAA GAG CCG GCT
[?]   [?]   [?]   [?]   [?]   [?]   [?]   N   E   E   I   [?]   G   I   [?]   [?]   [?]   [?]   [?]   [?]
1561                                                    1591
TTT GGA ATG GAC GCT TTA TGG ATT GAA ACT AAA AAT AAA GAA GAA GCC ATC ATT CAA GGC
F   G   M   D   A   L   W   I   E   T   K   N   K   E   E   A   I   I   Q   G
1621                                                    1651
TAT ACC ATT ATT GAT CCA AGC ACC GTT ATT GCG ACG CAC ACC AGC GAA TTA GTG AAA AAA
Y   T   I   I   D   P   S   T   V   I   A   T   H   T   S   E   L   V   K   K
1681                                                    1711
TAC GCT GAA GAT TTT ATC ACT AAA GAT GAA GTG AAA TCC CTT TTA GAG CGC TTG GCC AAA
Y   A   E   D   F   I   T   K   D   E   V   K   S   L   L   E   R   L   A   K
1741/581                                                1771
GAC TAT CCT ACG ATT GTA GAA GAG AGT AAA AAA ATC CCC ACC GGT GCG ATC CGA TCA GTG
D   Y   P   T   I   V   E   E   S   K   K   I   P   T   G   A   I   R   S   V
1801                                                    1831
TTG CAA GCC TTG TTG CAT GAA AAA ATC CCC ATT AAA GAC ATG CTC ACT ATT TTA GAA ACG
L   Q   A   L   L   H   E   K   I   P   I   K   D   M   L   T   I   L   E   T
1861                                                    1891
ATT ACC GAT ATT GCG CCA TTA GTT CAA AAC GAT GTG AAT ATC TTA ACC GAA CAA GTG AGG
I   T   D   I   A   P   L   V   Q   N   D   V   N   I   L   T   E   Q   V   R
1921                                                    1951
GCG AGG CTT TCT AGG GTG ATC ACT AAC GCT TTT AAA TCT GAA GAC GGG CGT TTG AAA TTT
A   R   L   S   R   V   I   T   N   A   F   K   S   E   D   G   R   L   K   F
1981                                                    2011
TTA ACC TTT TCT ACC GAT AGC GAA CAA TTT TTG CTT AAT AAA TTG CGA GAA AAT GGC ACT
L   T   F   S   T   D   S   E   Q   F   L   L   N   K   L   R   E   N   G   T
2041                                                    2071
TCT AAG AGC CTA CTA CTC AAT GTG GGC GAA TTG CAA AAA CTC ATT GAA GCG GTC TCT GAA
S   K   S   L   L   L   N   V   G   E   L   Q   K   L   I   E   A   V   S   E
2101                                                    2131
GAG GCC ATG AAA GTC TTG CAA AAA GGG ATC GCT CCG GTG ATT TTG ATC GTA GAG CCT AAT
E   A   M   K   V   L   Q   K   G   I   A   P   V   I   L   I   V   E   P   N
2161                                                    2191
TTA AGA AAA GCC CTT TCT AAT CAA ATG GAG CAG GCT AGG ATT GAT GTA ATC GTG CTA AGC
L   R   K   A   L   S   N   Q   M   E   Q   A   R   I   D   V   I   V   L   S
2221                                                    2251
CAT GCT GAA TTA GAT CCT AAC TCT AAC TTT GAA GCC TTA GGC ACG ATC CAT ATT AAC TTT
H   A   E   L   D   P   N   S   N   F   E   A   L   G   T   I   H   I   N   F
2281                                                    2311
TAA GGG ATA AAT AAT TGA TAA AAA AGG AGA ATG ATG CAA GTT TAT CAC CTT TCA CAC ATT
*
2341                                                    2371
GAT TTA GAC GGC TAT GCA TGC CAG CTT GTT TCA AAA CAA TTT TTT AAA AAT ATC CAA TGC
2401                                                    2431
TAT AAC GCT AAT TAC GGG CGT GAA GTC TCA GCG AGA ATT TAT GAG ATT TTA AAC GCG ATC
2461                                                    2491
GCT CAA TCT AAA GAG AGT GAA TTC CTT ATT TTG ATT AGC GA

3. Verwendung gemäß Anspruch 1 oder 2, wobei der rekombinante Bakterienstamm **dadurch gekennzeichnet ist, dass** ihm zusätzlich das Hakenprotein von H. pylori fehlt.

4. Rekombinante Bakterienstämme, die **dadurch gekennzeichnet sind, dass** sie durch Mutation, Substitution, Addition und/oder Deletion von Basen oder eines Nucleotidfragments in der Nucleotidsequenz des flbA-Gens, das an der Regulierung der Biosynthese der Flagellenproteine von H. pylori beteiligt ist, erhalten werden, wobei die Sequenz unter stringenten Bedingungen mit einer Sonde hybridisiert, die einem Nucleotidfragment von H. pylori entspricht, das mithilfe der beiden folgenden degenerierten Oligonucleotiden erweitert ist:
OLFlba-1 : ATGCCTCGAGGTCGAAAAGCAAGATG
und
OLFlba-2: GAAATCTTCATACTGGCAGCTCCAGTC,
wobei die Mutation so gestaltet ist, dass entweder das flbA-Gen nicht mehr im rekombinanten Bakterienstamm exprimiert wird, oder das flbA-Gen die Synthese der Flagellen von H. pylori nicht zulässt, und wobei die Mutation im Stamm N6 durchgeführt wird, der bei der NCIMB am 26. Juni 1992 unter der Nummer NCIMB 40512 hinterlegt wurde.

5. Rekombinanter Bakterienstamm gemäß Anspruch 4, **dadurch gekennzeichnet, dass** es sich um den Stamm N6flbA- handelt, der am 30. Juni 1995 unter der Nummer NCIMB 40747 bei der NCIMB hinterlegt wurde.

6. Verwendung gemäß einem der Ansprüche 1 bis 3, wobei der Bakterienstamm **dadurch gekennzeichnet ist**, das er zusätzlich so mutiert ist, dass er eine abgeschwächte Urease produziert, bzw. keine Urease mehr produziert, wobei die Mutation zum Beispiel in einer Mutation der Nucleotidsequenz eines oder mehrerer Gene besteht ausgewählt aus den Genen ureA, ureB, ureC, ureD, ureE, ureF, ureG, ureH oder ureI.

7. Rekombinanter Bakterienstamm gemäß Anspruch 4 oder 5, **dadurch gekennzeichnet, dass** er zusätzlich so mutiert ist, dass er eine abgeschwächte Urease produziert, bzw. keine Urease mehr produziert, wobei die Mutation zum Beispiel in einer Mutation der Nucleotidsequenz eines oder mehrerer Gene besteht ausgewählt aus den Genen ureA, ureB, ureC, ureD, ureE, ureF, ureG, ureH oder ureI.

8. Verwendung eines Bakterienextrakts zum *in vitro*-Nachweis einer Infektion mit H. pylori in einer Probe einer biologischen Flüssigkeit, die einem Patienten entnommen wurde, wobei der Bakterienextrakt **dadurch gekennzeichnet ist, dass** es sich um einen Extrakt eines Bakterienstammes handelt, der in einem der Ansprüche 1 bis 3 oder 6 definiert ist.

9. Verwendung gemäß Anspruch 8 eines Bakterienextrakts, wobei der Extrakt **dadurch gekennzeichnet ist, dass** er nach einer Extraktion mit N-Octylglukosid erhalten wird.

10. Verwendung gemäß Anspruch 8 eines Bakterienextrakts, wobei der Extrakt **dadurch gekennzeichnet ist, dass** er nach einer Extraktion mit PBS oder Glyzin erhalten wird.

11. Bakterienextrakt **dadurch gekennzeichnet, dass** es sich um einen Extrakt aus Bakterienstämmen gemäß einem der Ansprüche 4 oder 5 handelt.

12. Bakterienextrakt gemäß Anspruch 11, **dadurch gekennzeichnet, dass** er nach einer Extraktion mit N-Octylglucosid erhalten wird.

13. Bakterienextrakt gemäß Anspruch 11, **dadurch gekennzeichnet, dass** er nach einer Extraktion mit PBS oder Glyzin erhalten wird.

14. Verfahren zum in vitro-Nachweis einer Infektion mit H. pylori in einer Probe einer biologischen Flüssigkeit, die einem Patienten entnommen wurde, insbesondere in einer Serumprobe, das die Schritte umfasst:
- Inkontaktbringen der zu untersuchenden Probe mit einem in einem der Ansprüche 1 bis 6 definierten Bakterienstamm oder mit einem in einem der Ansprüche 8 bis 10 definierten Bakterienextrakt.
- Nachweis einer Immunreaktion zwischen dem Bakterienstamm und gegen H. pylori gerichteten Antikörpern, die in der zu untersuchenden Probe enthalten sind.

15. Verwendung eines in einem der Ansprüche 1 bis 6 definierten Bakterienstamms oder eines in einem der Ansprüche 8 bis 10 definierten Bakterienextrakts zum Erhalten von Antikörpern gegen H. pylori.

16. Verwendung gemäß Anspruch 15 für die Herstellung einer Impfzusammensetzung zum Erhalten von Antikörpern, die gegen eine Infektion mit H.pylori schützen.

17. Polyclonales Serum, das gegen einen Stamm von H. pylori gemäß einem der Ansprüche 4 oder 5 gerichtet ist.

18. Verwendung einer Zusammensetzung, die als aktives Prinzip ein polyclonales Serum enthält, das gegen einen in einem der Ansprüche 1 bis 6 definierten Stamm des flagellenlosen Phänotyps von H. pylori erhalten wurde, zum in vitro-Nachweis einer Infektion mit H**.** pylori in einer biologischen Probe.

19. Diagnostischer Kit von Antikörpern von mit H. pylori infizierten Patienten, der einen in einem der Ansprüche 1 bis 6 definierten Bakterienstamm oder einen in einem der Ansprüche 8 bis 10 definierten Bakterienextrakt sowie Reagenzien umfasst, die für den Nachweis einer Reaktion vom Typ Antigen-Antikörper erforderlich sind.
